(19) Europäisches Patentamt
European Patent Office
Office européen des brevets

(11) Veröffentlichungsnummer: **0 400 417 A1**

# (12) EUROPÄISCHE PATENTANMELDUNG

(21) Anmeldenummer: **90109441.7**

(51) Int. Cl.[5]: **C07C 251/40, A01N 37/50**

(22) Anmeldetag: **18.05.90**

(30) Priorität: **27.05.89 DE 3917352**

(43) Veröffentlichungstag der Anmeldung:
**05.12.90 Patentblatt 90/49**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Wenderoth, Bernd, Dr.**
**Schwalbenstrasse 26**
**D-6840 Lampertheim(DE)**
Erfinder: **Sauter, Hubert, Dr.**
**Neckarpromenade 20**
**D-6800 Mannheim 1(DE)**
Erfinder: **Wingert, Horst, Dr.**
**D 3,4,**
**D-6800 Mannheim 1(DE)**
Erfinder: **Hepp, Michael, Dr.**
**Im Steg 38**
**D-6802 Ladenburg(DE)**
Erfinder: **Brand, Siegbert Dr.**
**Hegelstrasse 39**
**D-6940 Weinheim(DE)**
Erfinder: **Kuekenhoehner, Thomas, Dr.**
**Seidelstrasse 2**
**D-6710 Frankenthal(DE)**
Erfinder: **Roehl, Franz, Dr.**
**Karl-Otto-Braun-Strasse 8**
**D-6700 Ludwigshafen(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

## (54) Neue Oximether und diese enthaltende Fungizide.

(57) Oximether der Formel

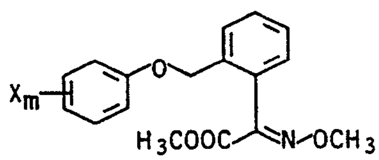

in der
X (m = 1 bis 5) gleiche oder verschiedene Substituenten Halogen, Cyano, Nitro, Alkyl, Cycloalkyl, Alkenyl, Alkoxy, Halogenalkyl, Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,
mit Ausnahme einiger bekannter Verbindungen und die neuen Verbindungen enthaltende Fungizide.

EP 0 400 417 A1

## Neue Oximether und diese enthaltende Fungizide

Die vorliegende Erfindung betrifft neue Oximether, ihre Herstellung, diese enthaltende Fungizide und ihre Verwendung als Fungizide.

Es ist bekannt, Oximether-Derivate wie zum Beispiel das 2-Phenoxymethylphe nylglyoxylsäuremethylester-O-methyloxim als Fungizide zu benutzen (EP 253 213, EP 254 426).

Es wurde nun gefunden, daß neue Oximether der allgemeinen Formel I

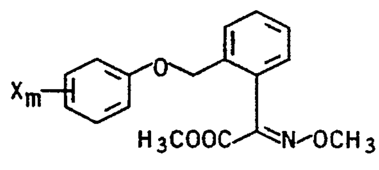

I

in der

x (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,

mit Ausnahme der Verbindungen, bei denen

$X_m$

2-Fluor-, 2-Chlor-, 3-Chlor-, 4-Chlor-, 2,4-Dichlor-, 2-Methyl-4-Chlor-, 2-Methyl-, 4-Methyl-, 4-tert.-Butyl-, 2-Methoxy-, 4-Methoxy-, 2-Trifluormethyl- oder 4-Nitro bedeutet, in ihrer fungiziden Wirkung den oben erwähnten bekannten Verbindungen überlegen sind.

Die in der allgemeinen Formel I aufgeführten Reste können beispielsweise folgende Bedeutung haben:

X (m = 1 bis 5, insbesondere 1 bis 3) kann zum Beispiel bedeuten:

Halogen (z.B. Fluor, Chlor, Brom, Jod), Cyano, Nitro, $C_1$-$C_{15}$-Alkyl ($C_1$-$C_{10}$-Alkyl) (z.B. Methyl, Ethyl, n- oder i-Propyl; n-, i-, s-oder t-Butyl; Pentyl, Hexyl, Heptyl, Octyl, Nonyl, Decyl), $C_3$-$C_6$-Cycloalkyl (z.B. Cyclopropyl, Cyclopentyl, Cyclohexyl), $C_3$-$C_6$-Alkenyl (z.B. 1-Propenyl, 2-Propenyl),$C_1$-$C_4$-Alkoxy (z.B. Methoxy, Ethoxy, n-Propoxy, i-Propoxy, n-Butoxy, t-Butoxy), $C_1$-$C_2$-Halogenalkyl (z.B. Difluormethyl, Trifluormethyl, Chlormethyl, Dichlormethyl, Trichlormethyl, Pentafluorethyl), $C_1$-$C_2$-Halogenalkoxy (z.B. Trifluormethoxy, Difluorethoxy, Tetrafluorethoxy, Pentafluorethoxy), gegebenenfalls substituiertes Phenyl (z.B. Phenyl, $C_1$-$C_4$-Alkylphenyl, Halogenphenyl), gegebenenfalls substituiertes Phenoxy (z.B. Phenoxy, $C_1$-$C_4$-Alkylphenoxy, Halogenphenoxy), gegebenenfalls substituiertes Benzyl (z.B. Benzyl, Halogenbenzyl) oder einen gegebenenfalls substituierten annellierten aromatischen Ring, der z.B. zu 1-Naphthyl- bzw. 2-Naphthylderivaten anstelle von Phenylderivaten führt.

Die neuen Verbindungen der allgemeinen Formel I können aufgrund der C=N-Doppelbindung sowohl als E- als auch als Z-Isomere vorliegen, die in üblicher Weise getrennt werden können. Sowohl die einzelnen isomeren Verbindungen als auch ihre Gemische werden von der Erfindung umfaßt und sind als Fungizide brauchbar.

Die neuen Verbindungen der allgemeinen Formel I lassen sich beispielsweise nach Schema 1 herstellen:

Schema 1:

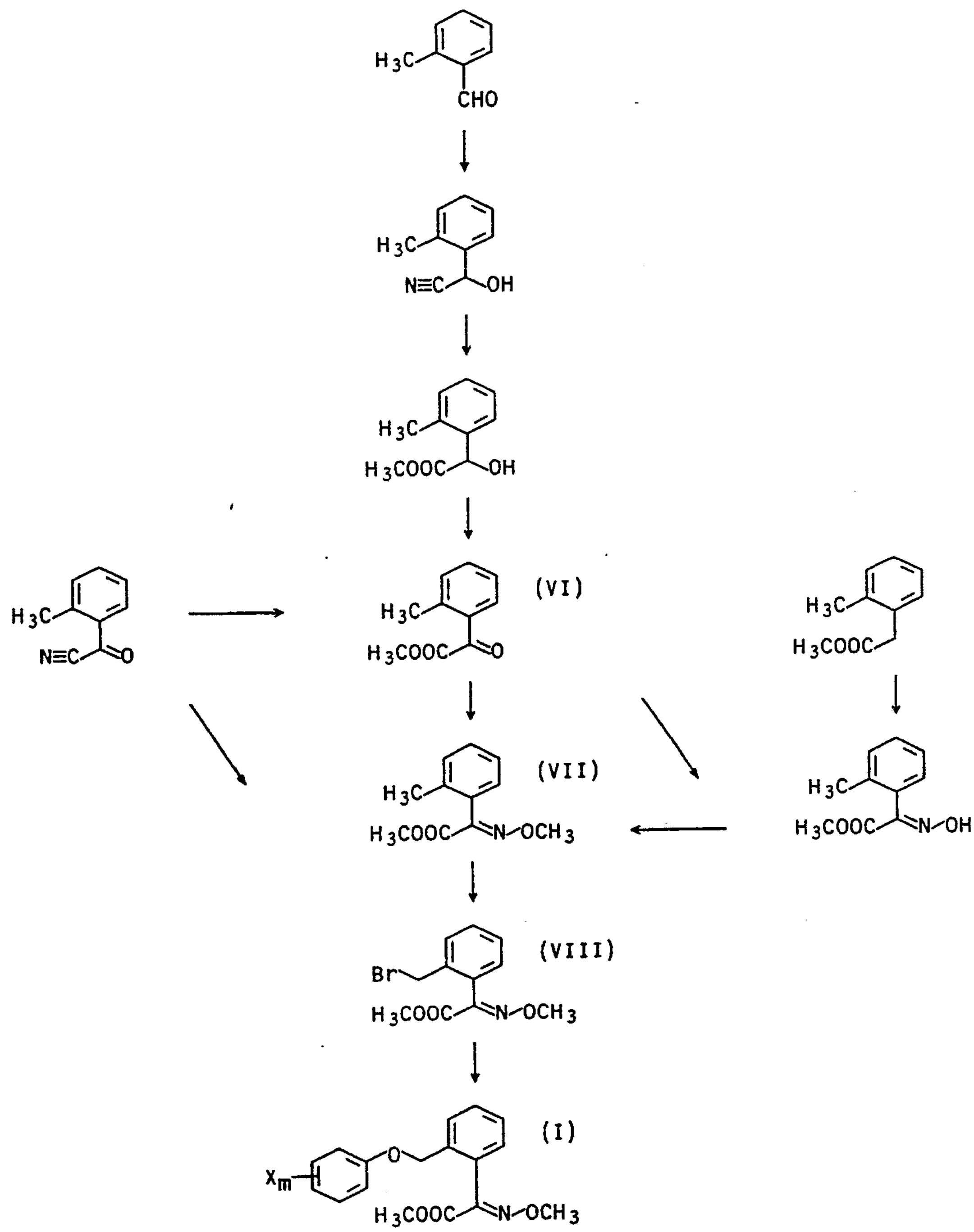

(VI)

(VII)

(VIII)

(I)

Der bekannte 2-Methylphenylglyoxylsäuremethylester VI (vgl. z.B. J. M. Photis, Tetrahedron Lett. 1980, 3539) läßt sich zum Beispiel herstellen, indem man 2-Methylbenzaldehyd mit Kalium- oder Natriumcyanid nach bekannten Verfahren in 2-Methylbenzaldehydcyanhydrin überführt, woraus sich in Gegenwart von Methanol und einer Protonsäure wie zum Beispiel Salzsäure 2-Methylmandelsäuremethylester herstellen läßt (vgl. z.B. US 2 892 847). Durch Umsetzung mit einem geeigneten Oxidationsmittel wie zum Beispiel Natriumhypochlorit, eventuell in Gegenwart eines entsprechenden Katalysators wie Tetrabutylammoniumhydrogensulfat, Tetrabutylammoniumbromid (vgl. z.B. EP 140 454) oder Tetramethylpiperidin-N-oxyd, erhält

man den Methylester VI, der auch aus 2-Methylbenzoylnitril durch Umsetzung mit Schwefelsäure/Methanol in Gegenwart von Natriumbromid gut zugänglich ist (vgl. z.B. J.M. Photis, Tetrahedron Lett. 1980, 3539).

2-Methylphenylglyoxylsäuremethylester-O-methyloxim VII läßt sich herstellen, indem man 2-Methylphenylglyoxylsäuremethylester VI z.B. a) mit O-Methylhydroxylamin-hydrochlorid umsetzt oder b) mit Hydroxylaminhydrochlorid zum entsprechenden Oxim umsetzt und dieses mit einem Methylierungsmittel der Formel $CH_3$-L, in der L eine Abgangsgruppe (z.B. Chlorid, Bromid, Jodid, Methylsulfat) bedeutet, zur Reaktion bringt (vgl. EP 253 213).

2-Methylphenylglyoxylsäuremethylester-O-methyloxim VII läßt sich weiterhin herstellen, indem man 2-Methylphenylessigsäuremethylester auf an sich bekannte Weise (vgl. z.B. Houben-Weyl, Methoden der organischen Chemie, Band 10/4, S. 28-32, Thieme Verlag, Stuttgart 1968) mit einem Alkylnitrit wie zum Beispiel Ethyl-, tert.-Butyl- oder Isoamylnitrit in Gegenwart einer geeigneten Base wie zum Beispiel Natrium-oder Kaliumethylat oder Kalium-tert.-butylat oximiert und das so erhaltene 2-Methylphenylglyoxylsäuremethylesteroxim wie oben beschrieben alkyliert.

Das Benzylbromid VIII läßt sich nach bekannten Methoden dadurch herstellen, daß man die Verbindung VII zum Beispiel mit Brom in einem Lösungsmittel wie zum Beispiel Tetrachlormethan, gegebenenfalls unter Belichtung mit einer Lichtquelle (z.B. Hg-Dampf-Lampe, 300 W) oder mit N-Bromsuccinimid umsetzt (Horner, Winkelmann, Angew. Chem. 71, 349 (1959)).

Die Darstellung der neuen Verbindungen der allgemeinen Formel I gemäß Anspruch 1 erfolgt beispielsweise so, daß man ein entsprechend substituiertes Phenol mit dem Benzylbromid VIII umsetzt.

Die Umsetzungen können z.B. in einem inerten Lösungs- oder Verdünnungsmittel (z.B. Aceton, Acetonitril, Dimethylsulfoxid, Dimethylformamid, N-Methylpyrrolidon, N,N′-Dimethylpropylenharnstoff oder Pyridin) unter Verwendung einer Base (z.B. Natriumcarbonat, Kaliumcarbonat) durchgeführt werden. Außerdem kann es von Vorteil sein, der Reaktionsmischung einen Katalysator wie z.B. Tris-(3,6-dioxoheptyl)-amin zuzusetzen.

Alternativ kann auch so vorgegangen werden, daß man die Phenole zunächst mit einer Base (z.B. Natriumhydroxid, Kaliumhydroxid, Natriummethanolat) in die entsprechenden Natrium- bzw. Kaliumphenolate überführt und diese dann in einem inerten Lösungs-oder Verdünnungsmittel (z.B. Dimethylformamid) mit dem Benzylbromid VIII zu den Verbindungen der allgemeinen Formel I umsetzt.

Die entsprechenden Umsetzungen können auch im Zweiphasensystem (z.B. Tetrachlorkohlenstoff/Wasser) durchgeführt werden. Als Phasentransfer-Katalysatoren kommen z.B. Trioctylpropylammoniumchlorid oder Cetyltrimethylammoniumchlorid in Betracht.

Die neuen Verbindungen der allgemeinen Formel I lassen sich weiterhin nach Schema 2 herstellen:

Schema 2:

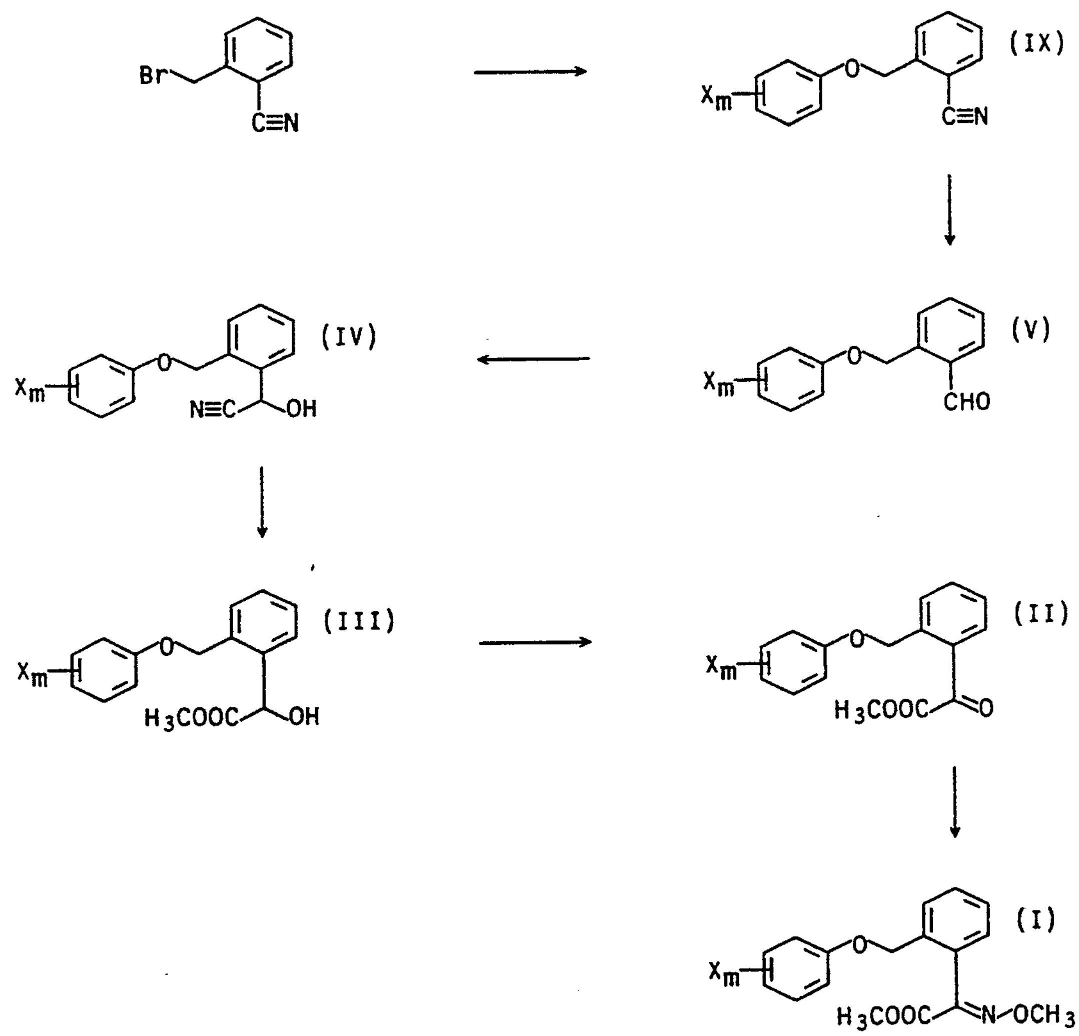

(IX) (IV) (V) (III) (II) (I)

2-(Brommethyl)-benzonitril wird mit einem entsprechend substituierten Phenol zu den Benzonitrilen der Formel IX umgesetzt.

Die Umsetzungen können z.B. in einem inerten Lösungs- oder Verdünnungsmittel (z.B. Aceton, Dimethylsulfoxid, Dimethylformamid, N-Methylpyrrolidon, N,N′-Dimethylpropylenharnstoff oder Pyridin) unter Verwendung einer Base (z.B. Natriumcarbonat, Kaliumcarbonat) durchgeführt werden. Außerdem kann es von Vorteil sein, der Reaktionsmischung einen Katalysator wie z.B. Tris-(3,6-dioxoheptyl)-amin zuzusetzen.

Alternativ kann auch so vorgegangen werden, daß man die Phenole zunächst mit einer Base (z.B. Natriumhydroxid, Kaliumhydroxid, Natriummethanolat) in die entsprechenden Natrium- bzw. Kaliumphenolate überführt und diese dann in einem inerten Lösungs-oder Verdünnungsmittel (z.B. Dimethylformamid) mit 2-(Brommethyl)-benzonitril umsetzt.

Die entsprechenden Umsetzungen können auch im Zweiphasensystem (z.B. Tetrachlorkohlenstoff/Wasser) durchgeführt werden. Als Phasentransfer-Katalysatoren kommen z.B. Trioctylpropylammoniumchlorid oder Cetyltrimethylammoniumchlorid in Betracht.

Die Benzonitrile der allgemeinen Formel IX werden zu den Benzaldehyd-Derivaten der allgemeinen Formel V reduziert.

Die Reduktion erfolgt zum Beispiel mit Salzsäure/Zinn(II)-chlorid (Stephen-Reduktion, vgl. z.B. Zil'berman, Pyryalova, J. Gen. Chem. USSR 33, 3348 (1964)) oder mit einem Metallhydrid wie zum Beispiel

Lithiumaluminiumhydrid oder Diisobutylaluminiumhydrid (vgl. z.B. Marshall, Andersen, Schlicher, J. Org. Chem. 35, 858 (1970)).

Die Verbindungen der allgemeinen Formel V sind neu und werden von dieser Erfindung ebenfalls umfaßt.

Aus den Aldehyden der allgemeinen Formel V lassen zum Beispiel mit Kalium-oder Natriumcyanid auf allgemein bekannte Weise Benzaldehydcyanhydrine der allgemeinen Formel IV herstellen.

Die Verbindungen der allgemeinen Formel IV sind neu und werden von dieser Erfindung ebenfalls umfaßt. In Gegenwart von Methanol und einer Protonsäure wie zum Beispiel Salzsäure lassen sie sich in die Mandelsäureester der allgemeinen Formel III überführen (vgl. z.B. US 2 892 847).

Die Verbindungen der allgemeinen Formel III sind neu und werden von dieser Erfindung ebenfalls umfaßt. Daraus erhält man durch Umsetzung mit einem geeigneten Oxidationsmittel wie zum Beispiel Natriumhypochlorit, eventuell in Gegenwart eines entsprechenden Katalysators wie Tetrabutylammoniumhydrogensulfat, Tetrabutylammoniumbromid (vgl. z.B. EP 140 454) oder Tetramethylpiperidin-N-oxid, alpha-Ketocarbonsäuremethylester der allgemeinen Formel II.

Die Verbindungen der allgemeinen Formel II sind neu und werden von dieser Erfindung ebenfalls umfaßt. Auf bekannte Weise (vgl. EP 253 213) lassen sich daraus die erfindungsgemäßen Verbindungen der allgemeinen Struktur I synthetisieren, indem man z.B. a) mit O-Methylhydroxylamin-hydrochlorid umsetzt oder b) mit Hydroxylamin zum entsprechenden Oxim umsetzt und dieses mit einem Methylierungsmittel der Formel $CH_3$-L, in der L eine Abgangsgruppe (z.B. Chlorid, Bromid, Methylsulfat) bedeutet, zur Reaktion bringt.

Die folgenden Beispiele und Vorschriften sollen die Herstellung der neuen Wirkstoffe und der neuen Zwischenprodukte erläutern.

Vorschrift 1

2-Methylbenzaldehydcyanhydrin

Zu einer Lösung von 245 g (3,75 mol) Kaliumcyanid und 201 g (3,75 mol) Ammoniumchlorid in 800 ml Wasser tropft man 240 g 2-Methylbenzaldehyd, gelöst in 1000 ml Methyl-tert.-Butylether.

Man rührt 5 Stunden bei Raumtemperatur und trennt die organische Phase ab. Die wässrige Phase wird 3 x mit Methyl-tert.-Butylether extrahiert. Die vereinigten organischen Phasen werden getrocknet und eingeengt.

275 g Rückstand (GC: 85 %) Ausbeute: 82 %.

Vorschrift 2:

2-Methylmandelsäuremethylester

275 g Rohprodukt aus Vorschrift 1 wird in 750 ml Methanol gelöst und zu 7,5 molarer methanolischer Salzsäure (620 ml) gegeben. Man rührt 15 h bei Raumtemperatur. Anschließend stellt man mit NaOH/$H_2O$ auf pH 3 und rührt 2 Stunden nach. Es wird mit Methylenchlorid extrahiert, getrocknet und eingeengt. Man erhält 280,5 g Rohprodukt (Gehalt an Endprodukt laut Gaschromatographie (GG) 76 %) Ausbeute: 76 %.

Vorschrift 3:

2-Methylphenylglyoxylsäuremethylester (VI)

a) 230 g Rohprodukt aus Vorschrift 2 wird in 230 g Essigester gelöst. Man gibt 13,6 g Tetrabutylammoniumhydrogensulfat zu. Unter Eiskühlung tropft man 3,45 Mol Natriumhypochloritlösung (NaOCl) zu. Es wird über Nacht bei Raumtemperatur gerührt. Man trennt die Essigesterphase ab, trocknet und engt ein. Man erhält 202 g Rohpodukt (GC 91 %) Ausbeute: 85 %.

b) Zu einer Mischung aus 1,73 g (0,017 mol) NaBr und 50 ml 85 %iger $H_2SO_4$ gibt man 30 g (0,168

mol) 2-Methylbenzoylcyanid. Man erhitzt auf 70 °C, gibt 100 ml Methanol zu und erhitzt weitere 3 Stunden bei dieser Temperatur. Nach dem Abkühlen wird mit Toluol extrahiert. Die organische Phase wird mit Wasser neutral gewaschen, getrocknet und eingeengt. Nach Destillation (92 °C, 0,1 mbar) erhält man 22 g (Ausbeute 73 %, GC: 87 %ig) Rohprodukt.

Vorschrift 4:

2-Methylphenylglyoxylsäuremethylester-O-methyloxim

a) 202 g Rohprodukt aus Vorschrift 3 wird in 1500 g Methanol gelöst. Man gibt 94 g 2-Methylhydroxylaminhydrochlorid zu und erhitzt 9 Stunden unter Rückfluß. Man nimmt in Essigester auf, trennt die organische Phase ab, trocknet und engt ein. Der Rückstand wird aus Methyl-tert.-Butylether/Hexan umkristallisiert.
(Ausbeute 65 %, Fp = 65-66 °C).

b) Zu 325 ml 85 %iger $H_2SO_4$ und 18,5 g Natriumbromid gibt man 195 g 2-Methylbenzoylcyanid, erhitzt auf 70 °C und tropft 650 ml Methanol zu. Das Reaktionsgemisch wird 3 h bei dieser Temperatur gerührt. Anschließend gibt man eine Lösung von 114 g 2-Methylhydroxylaminhydrochlorid in 2000 ml Methanol zu und erhitzt weitere 9 Stunden unter Rückfluß. Das Lösungsmittel wird entfernt, der Rückstand in Essigester aufgenommen, mit Wasser gewaschen, getrocknet und eingeengt. Man erhält 237 g (Ausbeute 85 %, GC 91 %) Produkt.

c) Zu 150 g (1,34 mol) Kalium-tert.-butylat in 1500 ml wasserfreiem tert.-Butanol gibt man zügig ein Gemisch aus 200 g (1,22 mol) 2-Methylphenylessigsäuremethylester und 377 g (3,66 mol) tert.-Butylnitrit in 400 ml tert.-Butanol, wobei die Reaktionstemperatur bis auf 70 °C ansteigt. Nach wenigen Minuten beginnt das sich bildende Kaliumsalz auszufallen, und die Reaktionslösung ist nur noch schwer rührbar. Es wird eine Stunde nachgerührt und dann auf einmal mit 261 g (1,84 mol) Methyljodid versetzt. Das Kaliumsalz geht langsam in Lösung und feinkristallines Kaliumjodid fällt aus. Man rührt über Nacht bei Raumtemperatur, engt ein, nimmt mit Methyl-tert.-butylether/$H_2O$ auf, wäscht mehrmals mit $H_2O$, trocknet über Natriumsulfat und engt erneut ein. Der erhaltene Rückstand wird warm in Isopropanol gelöst. Man gibt so lange $H_2O$ zu, bis die Trübung gerade noch verschwindet und läßt dann bei 0 °C auskristallisieren. Man erhält 114 g (45 %, Fp. = 65-66 °C) Produkt.

Vorschrift 5:

2-(Brommethyl)-phenylglyoxylsäuremethylester-O-methyloxim (VIII)

27,5 g (0,133 mol) 2-Methylphenylglyoxylsäuremethylester-O-methyloxim gelöst in 400 ml Tetrachlormethan werden unter Rühren mit 21,4 g (0,133 mol) Brom versetzt. Dann wird unter Bestrahlung mit einer 300 W-Hg-Dampf-Lampe vier Stunden auf Rückflußtemperatur erhitzt. Danach wird eingeengt, in Essigester/Wasser aufgenommen, mit $H_2O$ gewaschen, mit Natriumsulfat getrocknet und eingeengt. Das Rohprodukt wird mit Cyclohexan/Essigester (9/1) an Kieselgel chromatographisch gereinigt. Es werden 17,4 g (46 %) der oben genannten Verbindung als Öl erhalten.

Vorschrift 6:

2-(2,4-Dimethylphenoxymethyl)-benzonitril

382,5 g (2,65 mol) 2,4-Dimethylnatriumphenolat und 100 mg Kaliumjodid werden in 1,5 l N,N-Dimethylformamid gelöst. Portionsweise werden 400 g (2,04 mol) 2-Brommethylbenzonitril zugegeben. Es wird 16 h bei 40 °C gerührt. Dann wird eingeengt, in Essigester aufgenommen, mit $H_2O$ gewaschen, über Natriumsulfat getrocknet und eingeengt. Das Rohprodukt wird durch Verrühren mit wenig n-Penten gereinigt. Man erhält 340 g (70 %) als ockerfarbene Kristalle (Fp. = 75-76 °C)
$^1$H-NMR ($CDCl_3$): $\delta$ = 2.24(s,3H), 2.26(s,3H), 5.17(s,2H), 6.76(d,1H), 6.95(m,2H), 7.35(t,1H), 7.62(m,3H)

Vorschrift 7:

2-(2,4-Dimethylphenoxymethyl)-benzaldehyd (Tabelle 2, Nr. 2.62)

In 750 ml absolutem Toluol werden unter Stickstoff 100 g (0,42 mol) 2-(2,4-Dimethylphenoxymethyl)-benzonitril vorgelegt und bei 25 °C langsam mit 71 g (0,53 mol) einer 1,5 molaren Lösung von Diisobutylaluminiumhydrid versetzt. Nachdem 4 Stunden gerührt wurde, werden unter Kühlung im Eisbad zuerst 70 ml Methanol und danach 585 ml 10 %ige wäßrige HCl-Lösung zugetropft. Nachdem weitere 12 Stunden gerührt worden ist, wird die organische Phase abgetrennt, über Natriumsulfat getrocknet und eingeengt. Man erhält 100 g (99 %) als bräunliche Kristalle (Fp. = 70-71 °C)
$^1$H-NMR ($CDCl_3$): $\delta$ = 2.25(s,3H), 2.29(s,3H), 5.49(s,2H), 6.8-7.1(m,3H), 7.4-8.0(m,4H), 10.2(s,1H)

Vorschrift 8:

2-(2,4-Dimethylphenoxymethyl)-benzaldehydcyanhydrin (Tabelle 3, Nr. 3.62)

47,6 g (0,73 mol) Kaliumcyanid und 39,5 g (0,73 mol) Ammoniumchlorid werden in 220 ml $H_2O$ vorgelegt und bei 20 °C mit einer Lösung von 92 g (0,38 mol) 2-(2,4-Dimethylphenoxymethyl)-benzaldehyd in 425 ml Ether versetzt. Es wird 4 Tage gerührt und mit Ether gründlich ausgeschüttelt. Die vereinigten Etherphasen werden mit $H_2O$ gewaschen, über Natriumsulfat getrocknet und eingeengt. Man erhält 95 g Cyanhydrin als Rohprodukt (80 %ig), das ohne weitere Reinigung weiter umgesetzt wird.
$^1$H-NMR ($CDCl_3$): $\delta$ = 2.2(s,3H), 2.3(s,3H), 3.95(s,0H), 5.05(d,1H), 5.35(d,1H), 5.75(s,1H), 6.8-7.9(m,7H)

Vorschrift 9:

2-(2,4-Dimethylphenoxymethyl)-mandelsäuremethylester (Tabelle 4, Nr. 4.62)

0,3 mol 2-(2,4-Dimethylphenoxymethyl)-benzaldehydcyanhydrin und 0,6 mol Methanol werden in 850 ml absolutem Ether vorgelegt. Dazu tropft man bei 0 °C eine Lösung von 0,35 mol HCl-Gas in 250 ml absolutem Ether. Es wird ca. 12 Stunden bei 0 °C gerührt. Die ausgefallenen Kristalle werden abgetrennt, in 200 ml $H_2O$ gelöst und 15 min auf Rückflußtemperatur erhitzt. Nach dem Abkühlen wird mehrmals mit Ether extrahiert. Die vereinigten Etherphasen werden mit $H_2O$ gewaschen, über Natriumsulfat getrocknet und eingeengt. Das Rohprodukt wird über eine Flashsäule (Cyclohexan/Essigester = 8:2) gereinigt. Man erhält 7 g (7 %) als Öl.
1H-NMR ($CDCl_3$): $\delta$ = 2.25(s,3H), 2.30(s,3H), 3.5(s, ..H), 5.15(s,2H), 5.5(s,1H), 6.8-76.5(m,7H)

Vorschrift 10:

2-(2,4-Dimethylphenoxymethyl)-phenylglyoxylsäuremethylester (Tabelle 5, Nr. 5.62)

In einem Gemisch aus je 150 ml $H_2O$ und Dichlormethan werden 0,14 mol 2-(2,4-Dimethylphenoxymethyl)mandelsäuremethylester, 1,14 g N,N-Tetramethylpiperidin-N-oxyl, 0,8 g Kaliumbromid, 2,5 g $Na_2HPO_4$ x $2H_2O$ und 2,2 g $NaH_2PO_4$ x $2H_2O$ vorgelegt. Unter Rühren werden bei Raumtemperatur und pH 6-9 0,168 mol einer Natriumhypochlorit-Lösung in $H_2O$ zugetropft. Die wäßrige Phase wird 2 x mit Dichlormethan extrahiert. Die vereinigten organischen Phasen werden über Natriumsulfat getrocknet und eingeengt. Man erhält den gewünschten Glyoxylsäureester als Öl in quantitativer Ausbeute.
$^1$H-NMR ($CDCl_3$): $\delta$ = 2.6(s,6H), 3.85(s,3H), 5.35(s,2H), 6.7-7.0(m,3H), 7.3-7.8(m,4H)

Beispiel 1

2-(2,4-Dimethylphenoxymethyl)-phenylglyoxylsäuremethylester-O-methyloxim (Tabelle 1, Nr. 1.62)

86 g (0,6 mol) 2,4-Dimethyl-natriumphenolat und circa 0,5 g Kaliumjodid werden bei Raumtemperatur in 1000 ml N,N-Dimethylformamid vorgelegt und unter Rühren mit 172 g (0,6 mol) Brommethylverbindung VIII, gelöst in 300 ml N,N-Dimethylformamid, versetzt. Es wird 8 h bei 100 °C gerührt. Anschließend wird eingeengt. Der Rückstand wird in Essigester aufgenommen, mit $H_2O$ mehrmals gewaschen, über Natriumsulfat getrocknet und eingeengt. Das Rohprodukt wird mit n-Pentan aufgerührt, abgesaugt und getrocknet. Man erhält 125 g (64 %, Fp = 68-74 °C) der Verbindung als weiße Kristalle.

$^1$H-NMR ($CDCl_3$): $\delta$ = 2.22(s,3H), 2.24(s,3H), 3.83(s,3H), 4.05(s,3H), 4.95(s,2H), 6.60-7.60(m,7H)

Tabelle 1

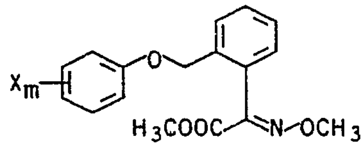

I

| Nr. | $X_m$ | Fp(°C) | IR($cm^{-1}$) |
|---|---|---|---|
| 1. 1 | 3-F | 72- 75 | 2945, 1728, 1593, 1490, 1220, 1135, 1070, 1020 |
| 1. 2 | 4-F | 74- 76 | 2970, 1733, 1726, 1508, 1222, 1204, 1070, 1016 |
| 1. 3 | 2,4-$F_2$ | 75- 76 | 2960, 1738, 1515, 1267, 1212, 1068, 1007, 768 |
| 1. 4 | 2,4,6-$F_3$ | | |
| 1. 5 | 2,3,4,5,6-$F_5$ | | |
| 1. 6 | 2,3-$F_2$ | | |
| 1. 7 | 2,3-$Cl_2$ | 105-108 | 2955, 1743, 1456, 1303, 1068, 1015, 769 |
| 1. 8 | 2,5-$Cl_2$ | 145-147 | 2945, 1738, 1583, 1379, 1301, 1248, 1069, 1009 |
| 1. 9 | 2,6-$Cl_2$ | 115-118 | 2945, 1729, 1444, 1250, 1061, 1009, 772 |
| 1. 10 | 3,4-$Cl_2$ | 90- 92 | 2950, 1740, 1591, 1468, 1300, 1225, 1070, 1013 |
| 1. 11 | 3,5-$Cl_2$ | 115-117 | 3080, 2945, 1743, 1587, 1573, 1306, 1070, 1008 |
| 1. 12 | 2,3,4-$Cl_3$ | | |
| 1. 13 | 2,3,5-$Cl_3$ | | |
| 1. 14 | 2,3,6-$Cl_3$ | | |
| 1. 15 | 2,4,5-$Cl_3$ | | |
| 1. 16 | 2,4,6-$Cl_3$ | | |
| 1. 17 | 3,4,5-$Cl_3$ | | |
| 1. 18 | 2,3,4,6-$Cl_4$ | | |
| 1. 19 | 2,3,5,6-$Cl_4$ | | |
| 1. 20 | 2,3,4,5,6-$Cl_5$ | | |
| 1. 21 | 2-Br | 94- 97 | 2950, 1729, 1479, 1241, 1065, 1018, 746 |
| 1. 22 | 3-Br | 66- 67 | 2945, 1738, 1595, 1307, 1245, 1228, 1069, 1010 |
| 1. 23 | 4-Br | 94- 96 | 2945, 1739, 1486, 1286, 1231, 1067, 1008, 824 |
| 1. 24 | 2,4-$Br_2$ | 106-108 | 2950, 1727, 1474, 1278, 1224, 1066, 1014 |
| 1. 25 | 2,5-$Br_2$ | | |
| 1. 26 | 2,6-$Br_2$ | | |
| 1. 27 | 2,4,6-$Br_3$ | | |
| 1. 28 | 2,3,4,5,6-$Br_5$ | | |
| 1. 29 | 2-J | 80- 83 | 2950, 1715, 1442, 1222, 1069, 1012, 744 |
| 1. 30 | 3-J | | |
| 1. 31 | 4-J | | |
| 1. 32 | 2,4-$J_2$ | | |
| 1. 33 | 2-Cl, 3-F | | |
| 1. 34 | 2-Cl, 4-F | 73- 75 | 2950, 1741, 1503, 1302, 1197, 1069, 1009, 791 |
| 1. 35 | 2-Cl, 5-F | | |

Tabelle 1 (Fortsetzung)

| Nr. | $X_m$ | Fp(°C) | IR($cm^{-1}$) |
|---|---|---|---|
| 1. 36 | 2-Cl, 6-F | | |
| 1. 37 | 2-Cl, 3-Br | | |
| 1. 38 | 2-Cl, 4-Br | 114-116 | 2945,1728,1479,1435,1264,1243,1067,1015 |
| 1. 39 | 2-Cl, 5-Br | | |
| 1. 40 | 2-Cl, 6-Br | | |
| 1. 41 | 2-Br, 3-Cl | | |
| 1. 42 | 2-Br, 4-Cl | 117-120 | 2940,1723,1479,1441,1218,1067,1021, 791 |
| 1. 43 | 2-Br, 3-F | | |
| 1. 45 | 2-Br, 4-F | 81- 84 | 2960,1725,1498,1434,1265,1197,1068,1013 |
| 1. 46 | 2-Br, 5-F | | |
| 1. 47 | 2-Br, 6-F | | |
| 1. 48 | 2-F, 3-Cl | | |
| 1. 49 | 2-F, 4-Cl | | |
| 1. 50 | 2-F, 5-Cl | | |
| 1. 51 | 3-Cl, 4-F | 86- 89 | 2945,1725,1499,1227,1213,1069,1011, 785 |
| 1. 52 | 3-Cl, 5-F | | |
| 1. 53 | 3-Cl, 4-Br | | |
| 1. 54 | 3-Cl, 5-Br | | |
| 1. 55 | 3-F, 4-Cl | | |
| 1. 56 | 3-F, 4-Br | | |
| 1. 57 | 3-Br, 4-Cl | | |
| 1. 58 | 3-Br, 4-F | | |
| 1. 59 | 2,6-$Cl_2$, 4-Br | | |
| 1. 60 | 3-$CH_3$ | 72 | 2950,1729,1256,1152,1066,1010, 779 |
| 1. 61 | 2,3-$(CH_3)_2$ | 110-113 | 2945,1740,1467,1302,1256,1062,1013, 771 |
| 1. 62 | 2,4-$(CH_3)_2$ | 68- 74 | 2945,1739,1504,1253,1223,1065,1005, 769 |
| 1. 63 | 2,5-$(CH_3)_2$ | 103-105 | |
| 1. 64 | 2,6-$(CH_3)_2$ | 85-88 | 1741,1438,1297,1230,1196,1068,1012,997, 773 |
| 1. 65 | 3,4-$(CH_3)_2$ | 89-93 | 1724,1502,1251,1225,1203,1163,1067,1030, 1012,954 |
| 1. 66 | 3,5-$(CH_3)_2$ | 80-83 | 1739,1594,1322,1302,1222,1152,1078,1065, 1009,825 |
| 1. 67 | 2,3,4-$(CH_3)_3$ | | |
| 1. 68 | 2,3,5-$(CH_3)_3$ | 97-98 | 2940,1740,1300,1219,1067,1008,774 |
| 1. 69 | 2,3,6-$(CH_3)_3$ | | |
| 1. 70 | 2,4,5-$(CH_3)_3$ | 94-95 | |
| 1. 71 | 2,4,6-$(CH_3)_3$ | 113-115 | |
| 1. 72 | 3,4,5-$(CH_3)_3$ | 94- 97 | 2960,1719,1441,1317,1223,1145,1065,1020 |

Tabelle 1 (Fortsetzung)

| Nr. | $X_m$ | Fp(°C) | IR($cm^{-1}$) |
|---|---|---|---|
| 1. 73 | 2,3,4,6-$(CH_3)_4$ | | |
| 1. 74 | 2,3,5,6-$(CH_3)_4$ | | |
| 1. 75 | 2,3,4,5,6-$(CH_3)_5$ | | |
| 1. 76 | 2-$C_2H_5$ | 46- 48 | 2960,1739,1493,1435,1222,1067,748 |
| 1. 77 | 3-$C_2H_5$ | | |
| 1. 78 | 4-$C_2H_5$ | | |
| 1. 79 | 2,4-$(C_2H_5)_2$ | | |
| 1. 80 | 2,6-$(C_2H_5)_2$ | | |
| 1. 81 | 3,5-$(C_2H_5)_2$ | öl | 2963,1727,1592,1455,1339,1285,1218,1152, 1069,1020 |
| 1. 82 | 2,4,6-$(C_2H_5)_3$ | | |
| 1. 83 | 2-n-$C_3H_7$ | 44- 46 | 2955,1727,1492,1321,1221,1069,1019,752 |
| 1. 84 | 3-n-$C_3H_7$ | | |
| 1. 85 | 4-n-$C_3H_7$ | | |
| 1. 86 | 2-i-$C_3H_7$ | öl | 2950,1727,1491,1233,1223,1069,1019,753 |
| 1. 87 | 3-i-$C_3H_7$ | | |
| 1. 88 | 4-i-$C_3H_7$ | öl | 2958,1728,1512,1222,1070,1019 |
| 1. 89 | 2,4-$(i\text{-}C_3H_7)_2$ | öl | 2956,1728,1498,1216,1069,1020 |
| 1. 90 | 2,6-$(i\text{-}C_3H_7)_2$ | | |
| 1. 91 | 3,5-$(i\text{-}C_3H_7)_2$ | öl | 2960,1728,1594,1447,1219,1069,1021 |
| 1. 92 | 2,4,6-$(i\text{-}C_3H_7)_3$ | | |
| 1. 93 | 2-s-$C_4H_9$ | öl | 2960,1727,1490,1448,1438,1221,1069,1047, 1019,752 |
| 1. 94 | 3-s-$C_4H_9$ | | |
| 1. 95 | 4-s-$C_4H_9$ | | |
| 1. 96 | 2-t-$C_4H_9$ | | |
| 1. 97 | 3-t-$C_4H_9$ | öl | 2962,1728,1582,1437,1274,1219,1069,1020 |
| 1. 98 | 2,3-$(t\text{-}C_4H_9)_2$ | | |
| 1. 99 | 2,4-$(t\text{-}C_4H_9)_2$ | | |
| 1.100 | 2,5-$(t\text{-}C_4H_9)_2$ | | |
| 1.101 | 2,6-$(t\text{-}C_4H_9)_2$ | | |
| 1.102 | 3,5-$(t\text{-}C_4H_9)_2$ | öl | 2963,1729,1592,1302,1219,1070,1020 |
| 1.103 | 2,4,6-$(t\text{-}C_4H_9)_3$ | | |
| 1.104 | 4-n-$C_9H_{19}$ | öl | 2956,2935,2871,1727,1510,1244,1220,1183, 1069,1019 |
| 1.105 | 4-n-$C_{12}H_{25}$ | | |
| 1.106 | 3-n-$C_{15}H_{31}$ | | |

Tabelle 1 (Fortsetzung)

| Nr. | $X_m$ | Fp(°C) | IR($cm^{-1}$) |
|---|---|---|---|
| 1.107 | 4-(1,1,3,3-Tetramethylbutyl) | öl | 2954,1728,1511,1317,1303,1245,1220,1183,1069,1020 |
| 1.108 | 4-(2,3,3-Trimethylpropyl) | | |
| 1.109 | 2-t-$C_4H_9$, 4-$CH_3$ | | |
| 1.110 | 2-t-$C_4H_9$, 5-$CH_3$ | | |
| 1.111 | 2,6-(t-$C_4H_9$)$_2$, 4-$CH_3$ | | |
| 1.112 | 2-$CH_3$, 4-t-$C_4H_9$ | | |
| 1.113 | 2-$CH_3$, 6-t-$C_4H_9$ | | |
| 1.114 | 2-$CH_3$, 4-i-$C_3H_7$ | | |
| 1.115 | 2-$CH_3$, 5-i-$C_3H_7$ | öl | 2957,1727,1437,1252,1218,1069,1020 |
| 1.116 | 3-$CH_3$, 4-i-$C_3H_7$ | | |
| 1.117 | 2-i-$C_3H_7$, 5-$CH_3$ | 70- 74 | 2958,1727,1505,1437,1290,1255,1219,1069,1045,1020 |
| 1.118 | 2,4-(t-$C_4H_9$)$_2$, 6-i-$C_3H_7$ | | |
| 1.119 | 2-$C_3H_5$ (= Allyl) | 47 | 2945,1739,1493,1304,1251,1069,1010,744 |
| 1.120 | 3-$C_3H_5$ | | |
| 1.121 | 4-$C_3H_5$ | | |
| 1.122 | 2-$C_3H_5$, 6-$CH_3$ | | |
| 1.123 | 2-cyclo-$C_6H_{11}$ | 115 | 2924,1726,1239,1228,1068,1016,750 |
| 1.124 | 3-cyclo-$C_6H_{11}$ | | |
| 1.125 | 4-cyclo-$C_6H_{11}$ | 93 | 2923,1731,1510,1239,1232,1063,1020,822 |
| 1.126 | 2,4-(cyclo-$C_6H_{11}$)$_2$, 6-$CH_3$ | | |
| 1.127 | 2-$CH_3$, 4-cyclo-$C_6H_{11}$ | öl | 2925,2851,1727,1504,1218,1070,1020 |
| 1.128 | 2-$CH_2C_6H_5$ | | |
| 1.129 | 3-$CH_2C_6H_5$ | | |
| 1.130 | 4-$CH_2C_6H_5$ | | |
| 1.131 | 2-$CH_2C_6H_5$, 4-$CH_3$ | öl | 1726,1501,1495,1452,1249,1220,1069,1019,731 |
| 1.132 | 2-$CH_3$, 4-$CH_2C_6H_5$ | | |
| 1.133 | 2-$C_6H_5$ | öl | 1739,1725,1481,1434,1263,1220,1069,1017,754,699 |

Tabelle 1 (Fortsetzung)

| Nr. | $X_m$ | Fp(°C) | IR($cm^{-1}$) |
|---|---|---|---|
| 1.134 | 3-$C_6H_5$ | Öl | 1726,1477,1320,1300,1219,1202,1069, 1017,759,698 |
| 1.135 | 4-$C_6H_5$ | Harz | 2955,1729,1519,1488,1245,1069,1015, 765 |
| 1.136 | 4-(2-i-$C_3H_7$-$C_6H_4$) | | |
| 1.137 | 4-$C_6H_5$, 2,6-$(CH_3)_2$ | 138-142 | 1739,1474,1305,1295,1229,11789,1068, 1010,1001,768 |
| 1.138 | 2-Cl, 4-$C_6H_5$ | Öl | 2945,1726,1596,1477,1301,1202,1069, 759 |
| 1.139 | 2-Br, 4-$C_6H_5$ | | |
| 1.140 | 2-$C_6H_5$, 4-Cl | | |
| 1.141 | 2-$C_6H_5$, 4-Br | | |
| 1.142 | 2-$CH_2C_6H_5$, 4-Cl | | |
| 1.143 | 2-$CH_2C_6H_5$, 4-Br | | |
| 1.144 | 2-Cl, 4-$CH_2C_6H_5$ | | |
| 1.145 | 2-Br, 4-$CH_2C_6H_5$ | | |
| 1.146 | 2-cyclo-$C_6H_{11}$, 4-Cl | | |
| 1.147 | 2-cyclo-$C_6H_{11}$, 4-Br | | |
| 1.148 | 2-Cl, 4-cyclo-$C_6H_{11}$ | | |
| 1.149 | 2-Br, 4-cyclo-$C_6H_{11}$ | | |
| 1.150 | 3-$OCH_3$ | 69- 71 | 2945,1719,1589,1200,1153,1069,1012, 756 |
| 1.151 | 2,4-$(OCH_3)_2$ | | |
| 1.152 | 2-$CF_3$ | | |
| 1.153 | 3-$CF_3$ | 46- 48 | 2955,1727,1719,1338,1323,1102,1067, 1014 |
| 1.154 | 4-$CF_3$ | | |
| 1.155 | 2-$OCF_3$ | | |
| 1.156 | 3-$OCF_3$ | | |
| 1.157 | 4-$OCF_3$ | Harz | 2950,1731,1506,1242,1222,1119,1069, 1017 |
| 1.158 | 3-$OCH_2CHF_2$ | | |
| 1.159 | 2-$NO_2$ | | |
| 1.160 | 3-$NO_2$ | Harz | |
| 1.161 | 2-CN | 103-105 | 2945,2230,1728,1450,1254,1062,1010, 762 |
| 1.162 | 3-CN | | |
| 1.163 | 4-CN | | |
| 1.164 | 2-$CH_3$, 3-Cl | | |

Tabelle 1 (Fortsetzung)

| Nr. | $X_m$ | Fp(°C) | IR($cm^{-1}$) |
|---|---|---|---|
| 1.165 | 2-$CH_3$, 5-Cl | | |
| 1.166 | 2-$CH_3$, 6-Cl | | |
| 1.167 | 2-$CH_3$, 3-F | | |
| 1.168 | 2-$CH_3$, 4-F | | |
| 1.169 | 2-$CH_3$, 5-F | | |
| 1.170 | 2-$CH_3$, 6-F | | |
| 1.171 | 2-$CH_3$, 3-Br | | |
| 1.172 | 2-$CH_3$, 4-Br | | |
| 1.173 | 2-$CH_3$, 5-Br | | |
| 1.174 | 2-$CH_3$, 6-Br | | |
| 1.175 | 2-Cl, 3-$CH_3$ | | |
| 1.176 | 2-Cl, 4-$CH_3$ | 90- 92 | 1726,1500,1437,1298,1285,1251,1218, 1068,1017 |
| 1.177 | 2-Cl, 5-$CH_3$ | 120-122 | 2940,1737,1489,1309,1070,1065,1008, 810 |
| 1.178 | 2-F, 3-$CH_3$ | | |
| 1.179 | 2-F, 4-$CH_3$ | | |
| 1.180 | 2-F, 5-$CH_3$ | | |
| 1.181 | 2-Br, 3-$CH_3$ | | |
| 1.182 | 2-Br, 4-$CH_3$ | | |
| 1.183 | 3-$CH_3$, 4-Cl | | |
| 1.184 | 3-$CH_3$, 5-Cl | | |
| 1.185 | 2-Br, 5-$CH_3$ | | |
| 1.186 | 3-$CH_3$, 4-F | | |
| 1.187 | 3-$CH_3$, 5-F | | |
| 1.188 | 3-$CH_3$, 4-Br | | |
| 1.189 | 3-$CH_3$, 5-Br | | |
| 1.190 | 3-F, 4-$CH_3$ | | |
| 1.191 | 3-Cl, 4-$CH_3$ | | |
| 1.192 | 3-Br, 4-$CH_3$ | | |
| 1.193 | 2-Cl, 4,5-$(CH_3)_2$ | | |
| 1.194 | 2-Br, 4,5-$(CH_3)_2$ | | |
| 1.195 | 2-Cl, 3,5-$(CH_3)_2$ | | |
| 1.196 | 2-Br, 3,5-$(CH_3)_2$ | | |
| 1.197 | 2,6-$Cl_2$, 4-$CH_3$ | | |
| 1.198 | 2,6-$F_2$, 4-$CH_3$ | | |
| 1.199 | 2,6-$Br_2$, 4-$CH_3$ | | |
| 1.200 | 2,4-$Cl_2$, 6-$CH_3$ | | |
| 1.201 | 2,4-$F_2$, 6-$CH_3$ | | |

Tabelle 1 (Fortsetzung)

| Nr. | $X_m$ | Fp(°C) | IR($cm^{-1}$) |
|---|---|---|---|
| 1.202 | 2,4-$Br_2$, 6-$CH_3$ | | |
| 1.203 | 2,6-$(CH_3)_2$, 4-F | | |
| 1.204 | 2,6-$(CH_3)_2$, 4-Cl | 94- 97 | 2950,1721,1441,1323,1220,1200,1065, 1013 |
| 1.205 | 2,6-$(CH_3)_2$, 4-Br | Harz | 2950,1722,1436,1321,1220,1199,1067, 1014 |
| 1.206 | 3,5-$(CH_3)_2$, 4-F | | |
| 1.207 | 3,5-$(CH_3)_2$, 4-Cl | | |
| 1.208 | 3,5-$(CH_3)_2$, 4-Br | | |
| 1.209 | 2,3,6-$(CH_3)_3$, 4-F | | |
| 1.210 | 2,3,6-$(CH_3)_3$, 4-Cl | | |
| 1.211 | 2,3,6-$(CH_3)_3$, 4-Br | | |
| 1.212 | 2,4-$(CH_3)_2$, 6-F | | |
| 1.213 | 2,4-$(CH_3)_2$, 6-Cl | | |
| 1.214 | 2,4-$(CH_3)_2$, 6-Br | | |
| 1.215 | 2-i-$C_3H_7$, 4-Cl, 5-$CH_3$ | Öl | 2960,1727,1495,1437,1245,1219,1170, 1124,1070,1019 |
| 1.216 | 2-Cl, 4-$NO_2$ | | |
| 1.217 | 2-$NO_2$, 4-Cl | | |
| 1.218 | 2-$OCH_3$, 5-$NO_2$ | | |
| 1.219 | 2,4-$Cl_2$, 5-$NO_2$ | | |
| 1.220 | 2,4-$Cl_2$, 6-$NO_2$ | | |
| 1.221 | 2,6-$Cl_2$, 4-$NO_2$ | | |
| 1.222 | 2,6-$Br_2$, 4-$NO_2$ | | |
| 1.223 | 2,6-$J_2$, 4-$NO_2$ | | |
| 1.224 | 2-$CH_3$, 5-i-$C_3H_7$, 4-Cl | Öl | 2962,1727,1492,1437,1255,1217,1165, 1069,1047,1020 |
| 1.225 | 2-$C_6H_5O$ | | |
| 1.226 | 3-$C_6H_5O$ | | |
| 1.227 | 4-$C_6H_5O$ | | |
| 1.228 | 3-t-$C_4H_9O$ | | |
| 1.229 | 4-t-$C_4H_9O$ | | |

Tabelle 1 (Fortsetzung)

| Nr. | $X_m$ | Fp(°C) | IR($cm^{-1}$) |
|---|---|---|---|
| 1.230 | *1) | öl | 2945, 1726, 1400, 1269, 1097, 1069, 1019, 771 |
| 1.231 | *2) | öl | 2950, 1727, 1441, 1214, 1177, 1066, 1012, 956 |
| 1.232 | 4-$OCH_2C_6H_5$ | 130-133 | 2955, 1728, 1504, 1231, 1204, 1069, 1016, 741 |
| 1.233 | 2-$OC_2H_5$ | | |
| 1.234 | 2-$CH_3$, 4-(1,1,3,3,-Tetramethylbutyl) | öl | 2952, 2901, 1728, 1507, 1259, 1243, 1219, 1069, 1020 |
| 1.235 | 2-Cl, 3-i-$C_3H_7$ | | |
| 1.236 | 2-$CH_3$, 4-$C_6H_5$, | | |
| 1.237 | 4-(1,1,3-Trimethylbutyl) | | |
| 1.238 | 3-$CH_3$, 5-i-$C_3H_7$ | öl | 2958, 1727, 1593, 1334, 1322, 1291, 1218, 1069, 1045, 1020 |
| 1.239 | 2-$CH_3$, 4-Cyclo-$C_5H_9$ | | |
| 1.240 | 3-n-$C_4H_9O$ | öl | 1727, 1591, 1492, 1284, 1219, 1179, 1152, 1069, 1044, 1019 |
| 1.241 | 4-n-$C_4H_9O$ | 66- 67 | |
| 1.242 | 3-n-$C_6H_{13}O$ | öl | 2935, 1727, 1591, 1492, 1218, 1178, 1152, 1069, 1045, 1019 |
| 1.243 | 4-n-$C_6H_{13}O$ | 69- 70 | |
| 1.244 | 4-$OC_2H_5$ | 64- 66 | 1722, 1512, 1438, 1241, 1216, 1118, 1069, 1047, 1030 |
| 1.245 | 2-$OCH_3$, 4-$CH_3$ | öl | 1727, 1512, 1464, 1301, 1265, 1221, 1157, 1141, 1069, 1018 |
| 1.246 | *3) | 82- 86 | |

*1)

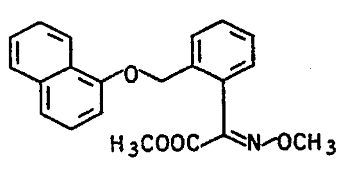

*2)

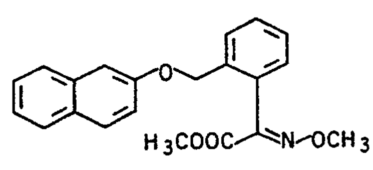

*3)

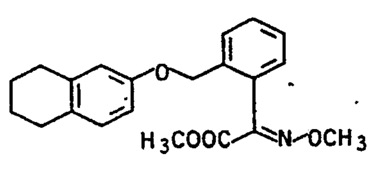

Tabelle 2

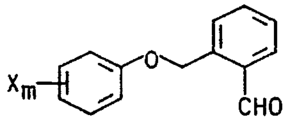

(V)

| Nr. | $X_m$ | Fp(°C) | IR($cm^{-1}$) |
|---|---|---|---|
| 2. 1 | 3-F | | |
| 2. 2 | 4-F | | |
| 2. 3 | 2,4-$F_2$ | | |
| 2. 4 | 2,4,6-$F_3$ | | |
| 2. 5 | 2,3,4,5,6-$F_5$ | | |
| 2. 6 | 2,3-$F_2$ | | |
| 2. 7 | 2,3-$Cl_2$ | | |
| 2. 8 | 2,5-$Cl_2$ | | |
| 2. 9 | 2,6-$Cl_2$ | | |
| 2. 10 | 3,4-$Cl_2$ | | |
| 2. 11 | 3,5-$Cl_2$ | | |
| 2. 12 | 2,3,4-$Cl_3$ | | |
| 2. 13 | 2,3,5-$Cl_3$ | | |
| 2. 14 | 2,3,6-$Cl_3$ | | |
| 2. 15 | 2,4,5-$Cl_3$ | | |
| 2. 16 | 2,4,6-$Cl_3$ | | |
| 2. 17 | 3,4,5-$Cl_3$ | | |
| 2. 18 | 2,3,4,6-$Cl_4$ | | |
| 2. 19 | 2,3,5,6-$Cl_4$ | | |
| 2. 20 | 2,3,4,5,6-$Cl_5$ | | |
| 2. 21 | 2-Br | | |
| 2. 22 | 3-Br | | |
| 2. 23 | 4-Br | | |
| 2. 24 | 2,4-$Br_2$ | | |
| 2. 25 | 2,5-$Br_2$ | | |
| 2. 26 | 2,6-$Br_2$ | | |
| 2. 27 | 2,4,6-$Br_3$ | | |
| 2. 28 | 2,3,4,5,6-$Br_5$ | | |
| 2. 29 | 2-J | | |
| 2. 30 | 3-J | | |
| 2. 31 | 4-J | | |
| 2. 32 | 2,4-$J_2$ | | |
| 2. 33 | 2-Cl, 3-F | | |
| 2. 34 | 2-Cl, 4-F | | |
| 2. 35 | 2-Cl, 5-F | | |

Tabelle 2 (Fortsetzung)

| Nr. | $X_m$ | Fp(°C) | IR($cm^{-1}$) |
|---|---|---|---|
| 2. 36 | 2-Cl, 6-F | | |
| 2. 37 | 2-Cl, 3-Br | | |
| 2. 38 | 2-Cl, 4-Br | | |
| 2. 39 | 2-Cl, 5-Br | | |
| 2. 40 | 2-Cl, 6-Br | | |
| 2. 41 | 2-Br, 3-Cl | | |
| 2. 42 | 2-Br, 4-Cl | | |
| 2. 43 | 2-Br, 3-F | | |
| 2. 45 | 2-Br, 4-F | | |
| 2. 46 | 2-Br, 5-F | | |
| 2. 47 | 2-Br, 6-F | | |
| 2. 48 | 2-F, 3-Cl | | |
| 2. 49 | 2-F, 4-Cl | | |
| 2. 50 | 2-F, 5-Cl | | |
| 2. 51 | 3-Cl, 4-F | | |
| 2. 52 | 3-Cl, 5-F | | |
| 2. 53 | 3-Cl, 4-Br | | |
| 2. 54 | 3-Cl, 5-Br | | |
| 2. 55 | 3-F, 4-Cl | | |
| 2. 56 | 3-F, 4-Br | | |
| 2. 57 | 3-Br, 4-Cl | | |
| 2. 58 | 3-Br, 4-F | | |
| 2. 59 | 2,6-$Cl_2$, 4-Br | | |
| 2. 60 | 3-$CH_3$ | | |
| 2. 61 | 2,3-$(CH_3)_2$ | | |
| 2. 62 | 2,4-$(CH_3)_2$ | 70- 71 | 1855,1685,1502,1254,1223,1131,1033, 805,764 |
| 2. 63 | 2,5-$(CH_3)_2$ | | |
| 2. 64 | 2,6-$(CH_3)_2$ | | |
| 2. 65 | 3,4-$(CH_3)_2$ | | |
| 2. 66 | 3,5-$(CH_3)_2$ | | |
| 2. 67 | 2,3,4-$(CH_3)_3$ | | |
| 2. 68 | 2,3,5-$(CH_3)_3$ | | |
| 2. 69 | 2,3,6-$(CH_3)_3$ | | |
| 2. 70 | 2,4,5-$(CH_3)_3$ | | |
| 2. 71 | 2,4,6-$(CH_3)_3$ | | |
| 2. 72 | 3,4,5-$(CH_3)_3$ | | |
| 2. 73 | 2,3,4,6-$(CH_3)_4$ | | |
| 2. 74 | 2,3,5,6-$(CH_3)_4$ | | |

Tabelle 2 (Fortsetzung)

| Nr. | $X_m$ | Fp(°C) | IR($cm^{-1}$) |
|---|---|---|---|
| 2. 75 | 2,3,4,5,6-$(CH_3)_5$ | | |
| 2. 76 | 2-$C_2H_5$ | | |
| 2. 77 | 3-$C_2H_5$ | | |
| 2. 78 | 4-$C_2H_5$ | | |
| 2. 79 | 2,4-$(C_2H_5)_2$ | | |
| 2. 80 | 2,6-$(C_2H_5)_2$ | | |
| 2. 81 | 3,5-$(C_2H_5)_2$ | | |
| 2. 82 | 2,4,6-$(C_2H_5)_3$ | | |
| 2. 83 | 2-n-$C_3H_7$ | | |
| 2. 84 | 3-n-$C_3H_7$ | | |
| 2. 85 | 4-n-$C_3H_7$ | | |
| 2. 86 | 2-i-$C_3H_7$ | | |
| 2. 87 | 3-i-$C_3H_7$ | | |
| 2. 88 | 4-i-$C_3H_7$ | | |
| 2. 89 | 2,4-$(i\text{-}C_3H_7)_2$ | | |
| 2. 90 | 2,6-$(i\text{-}C_3H_7)_2$ | | |
| 2. 91 | 3,5-$(i\text{-}C_3H_7)_2$ | | |
| 2. 92 | 2,4,6-$(i\text{-}C_3H_7)_3$ | | |
| 2. 93 | 2-s-$C_4H_9$ | | |
| 2. 94 | 3-s-$C_4H_9$ | | |
| 2. 95 | 4-s-$C_4H_9$ | | |
| 2. 96 | 2-t-$C_4H_9$ | | |
| 2. 97 | 3-t-$C_4H_9$ | | |
| 2. 98 | 2,3-$(t\text{-}C_4H_9)_2$ | | |
| 2. 99 | 2,4-$(t\text{-}C_4H_9)_2$ | | |
| 2.100 | 2,5-$(t\text{-}C_4H_9)_2$ | | |
| 2.101 | 2,6-$(t\text{-}C_4H_9)_2$ | | |
| 2.102 | 3,5-$(t\text{-}C_4H_9)_2$ | | |
| 2.103 | 2,4,6-$(t\text{-}C_4H_9)_3$ | | |
| 2.104 | 4-n-$C_9H_{19}$ | | |
| 2.105 | 4-n-$C_{12}H_{25}$ | | |
| 2.106 | 3-n-$C_{15}H_{31}$ | | |
| 2.107 | 4-(1,1,3,3-Tetramethylbutyl) | | |
| 2.108 | 4-(2,3,3-Trimethylpropyl) | | |
| 2.109 | 2-t-$C_4H_9$, 4-$CH_3$ | | |
| 2.110 | 2-t-$C_4H_9$, 5-$CH_3$ | | |
| 2.111 | 2,6-$(t\text{-}C_4H_9)_2$, 4-$CH_3$ | | |
| 2.112 | 2-$CH_3$, 4-t-$C_4H_9$ | | |
| 2.113 | 2-$CH_3$, 6-t-$C_4H_9$ | | |

Tabelle 2 (Fortsetzung)

| Nr. | $X_m$ | Fp(°C) | IR($cm^{-1}$) |
|---|---|---|---|
| 2.114 | 2-$CH_3$, 4-i-$C_3H_7$ | | |
| 2.115 | 2-$CH_3$, 5-i-$C_3H_7$ | | |
| 2.116 | 3-$CH_3$, 4-i-$C_3H_7$ | | |
| 2.117 | 2-i-$C_3H_7$, 5-$CH_3$ | | |
| 2.118 | 2,4-(t-$C_4H_9$)$_2$, 6-i-$C_3H_7$ | | |
| 2.119 | 2-$C_3H_5$ (= Allyl) | | |
| 2.120 | 3-$C_3H_5$ | | |
| 2.121 | 4-$C_3H_5$ | | |
| 2.122 | 2-$C_3H_5$, 6-$CH_3$ | | |
| 2.123 | 2-cyclo-$C_6H_{11}$ | | |
| 2.124 | 3-cyclo-$C_6H_{11}$ | | |
| 2.125 | 4-cyclo-$C_6H_{11}$ | | |
| 2.126 | 2,4-(cyclo-$C_6H_{11}$)$_2$, 6-$CH_3$ | | |
| 2.127 | 2-$CH_3$, 4-cyclo-$C_6H_{11}$ | | |
| 2.128 | 2-$CH_2C_6H_5$ | | |
| 2.129 | 3-$CH_2C_6H_5$ | | |
| 2.130 | 4-$CH_2C_6H_5$ | | |
| 2.131 | 2-$CH_2C_6H_5$, 4-$CH_3$ | | |
| 2.132 | 2-$CH_3$, 4-$CH_2C_6H_5$ | | |
| 2.133 | 2-$C_6H_5$ | | |
| 2.134 | 3-$C_6H_5$ | | |
| 2.135 | 4-$C_6H_5$ | | |
| 2.136 | 4-(2-i-$C_3H_7$-$C_6H_4$) | | |
| 2.137 | 4-$C_6H_5$, 2,6-($CH_3$)$_2$ | | |
| 2.138 | 2-Cl, 4-$C_6H_5$ | | |
| 2.139 | 2-Br, 4-$C_6H_5$ | | |
| 2.140 | 2-$C_6H_5$, 4-Cl | | |
| 2.141 | 2-$C_6H_5$, 4-Br | | |
| 2.142 | 2-$CH_2C_6H_5$, 4-Cl | | |
| 2.143 | 2-$CH_2C_6H_5$, 4-Br | | |
| 2.144 | 2-Cl, 4-$CH_2C_6H_5$ | | |
| 2.145 | 2-Br, 4-$CH_2C_6H_5$ | | |
| 2.146 | 2-cyclo-$C_6H_{11}$, 4-Cl | | |
| 2.147 | 2-cyclo-$C_6H_{11}$, 4-Br | | |
| 2.148 | 2-Cl, 4-cyclo-$C_6H_{11}$ | | |
| 2.149 | 2-Br, 4-cyclo-$C_6H_{11}$ | | |
| 2.150 | 3-$OCH_3$ | | |
| 2.151 | 2,4-($OCH_3$)$_2$ | | |
| 2.152 | 2-$CF_3$ | | |

Tabelle 2 (Fortsetzung)

| Nr. | $X_m$ | Fp($^oC$) | IR($cm^{-1}$) |
|---|---|---|---|
| 2.153 | 3-$CF_3$ | | |
| 2.154 | 4-$CF_3$ | | |
| 2.155 | 2-$OCF_3$ | | |
| 2.156 | 3-$OCF_3$ | | |
| 2.157 | 4-$OCF_3$ | | |
| 2.158 | 3-$OCH_2CHF_2$ | | |
| 2.159 | 2-$NO_2$ | | |
| 2.160 | 3-$NO_2$ | | |
| 2.161 | 2-CN | | |
| 2.162 | 3-CN | | |
| 2.163 | 4-CN | | |
| 2.164 | 2-$CH_3$, 3-Cl | | |
| 2.165 | 2-$CH_3$, 5-Cl | | |
| 2.166 | 2-$CH_3$, 6-Cl | | |
| 2.167 | 2-$CH_3$, 3-F | | |
| 2.168 | 2-$CH_3$, 4-F | | |
| 2.169 | 2-$CH_3$, 5-F | | |
| 2.170 | 2-$CH_3$, 6-F | | |
| 2.171 | 2-$CH_3$, 3-Br | | |
| 2.172 | 2-$CH_3$, 4-Br | | |
| 2.173 | 2-$CH_3$, 5-Br | | |
| 2.174 | 2-$CH_3$, 6-Br | | |
| 2.175 | 2-Cl, 3-$CH_3$ | | |
| 2.176 | 2-Cl, 4-$CH_3$ | | |
| 2.177 | 2-Cl, 5-$CH_3$ | | |
| 2.178 | 2-F, 3-$CH_3$ | | |
| 2.179 | 2-F, 4-$CH_3$ | | |
| 2.180 | 2-F, 5-$CH_3$ | | |
| 2.181 | 2-Br, 3-$CH_3$ | | |
| 2.182 | 2-Br, 4-$CH_3$ | | |
| 2.183 | 3-$CH_3$, 4-Cl | | |
| 2.184 | 3-$CH_3$, 5-Cl | | |
| 2.185 | 2-Br, 5-$CH_3$ | | |
| 2.186 | 3-$CH_3$, 4-F | | |
| 2.187 | 3-$CH_3$, 5-F | | |
| 2.188 | 3-$CH_3$, 4-Br | | |
| 2.189 | 3-$CH_3$, 5-Br | | |
| 2.190 | 3-F, 4-$CH_3$ | | |
| 2.191 | 3-Cl, 4-$CH_3$ | | |

Tabelle 2 (Fortsetzung)

| Nr. | $X_m$ | Fp($^oC$) | IR($cm^{-1}$) |
|---|---|---|---|
| 2.192 | 3-Br, 4-$CH_3$ | | |
| 2.193 | 2-Cl, 4,5-$(CH_3)_2$ | | |
| 2.194 | 2-Br, 4,5-$(CH_3)_2$ | | |
| 2.195 | 2-Cl, 3,5-$(CH_3)_2$ | | |
| 2.196 | 2-Br, 3,5-$(CH_3)_2$ | | |
| 2.197 | 2,6-$Cl_2$, 4-$CH_3$ | | |
| 2.198 | 2,6-$F_2$, 4-$CH_3$ | | |
| 2.199 | 2,6-$Br_2$, 4-$CH_3$ | | |
| 2.200 | 2,4-$Cl_2$, 6-$CH_3$ | | |
| 2.201 | 2,4-$F_2$, 6-$CH_3$ | | |
| 2.202 | 2,4-$Br_2$, 6-$CH_3$ | | |
| 2.203 | 2,6-$(CH_3)_2$, 4-F | | |
| 2.204 | 2,6-$(CH_3)_2$, 4-Cl | | |
| 2.205 | 2,6-$(CH_3)_2$, 4-Br | | |
| 2.206 | 3,5-$(CH_3)_2$, 4-F | | |
| 2.207 | 3,5-$(CH_3)_2$, 4-Cl | | |
| 2.208 | 3,5-$(CH_3)_2$, 4-Br | | |
| 2.209 | 2,3,6-$(CH_3)_3$, 4-F | | |
| 2.210 | 2,3,6-$(CH_3)_3$, 4-Cl | | |
| 2.211 | 2,3,6-$(CH_3)_3$, 4-Br | | |
| 2.212 | 2,4-$(CH_3)_2$, 6-F | | |
| 2.213 | 2,4-$(CH_3)_2$, 6-Cl | | |
| 2.214 | 2,4-$(CH_3)_2$, 6-Br | | |
| 2.215 | 2-i-$C_3H_7$, 4-Cl, 5-$CH_3$ | | |
| 2.216 | 2-Cl, 4-$NO_2$ | | |
| 2.217 | 2-$NO_2$, 4-Cl | | |
| 2.218 | 2-$OCH_3$, 5-$NO_2$ | | |
| 2.219 | 2,4-$Cl_2$, 5-$NO_2$ | | |
| 2.220 | 2,4-$Cl_2$, 6-$NO_2$ | | |
| 2.221 | 2,6-$Cl_2$, 4-$NO_2$ | | |
| 2.222 | 2,6-$Br_2$, 4-$NO_2$ | | |
| 2.223 | 2,6-$J_2$, 4-$NO_2$ | | |
| 2.224 | 2-$CH_3$, 5-i-$C_3H_7$, 4-Cl | | |
| 2.225 | 2-$C_6H_5O$ | | |
| 2.226 | 3-$C_6H_5O$ | | |
| 2.227 | 4-$C_6H_5O$ | | |
| 2.228 | 3-t-$C_4H_9O$ | | |
| 2.229 | 4-t-$C_4H_9O$ | | |

Tabelle 2 (Fortsetzung)

| Nr. | $X_m$ | Fp(°C) | IR($cm^{-1}$) |
|---|---|---|---|
| 2.230 | *1) | | |
| 2.231 | *2) | | |

*1) 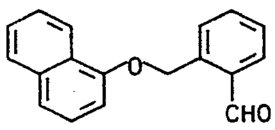

*2) 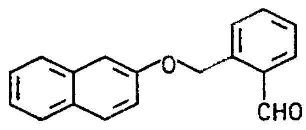

Tabelle 3

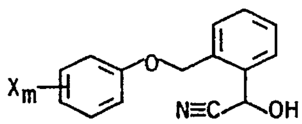

(IV)

| Nr. | $X_m$ | Fp(°C) | IR($cm^{-1}$) |
|---|---|---|---|
| 3. 1 | 3-F | | |
| 3. 2 | 4-F | | |
| 3. 3 | 2,4-$F_2$ | | |
| 3. 4 | 2,4,6-$F_3$ | | |
| 3. 5 | 2,3,4,5,6-$F_5$ | | |
| 3. 6 | 2,3-$F_2$ | | |
| 3. 7 | 2,3-$Cl_2$ | | |
| 3. 8 | 2,5-$Cl_2$ | | |
| 3. 9 | 2,6-$Cl_2$ | | |
| 3. 10 | 3,4-$Cl_2$ | | |
| 3. 11 | 3,5-$Cl_2$ | | |
| 3. 12 | 2,3,4-$Cl_3$ | | |
| 3. 13 | 2,3,5-$Cl_3$ | | |
| 3. 14 | 2,3,6-$Cl_3$ | | |
| 3. 15 | 2,4,5-$Cl_3$ | | |
| 3. 16 | 2,4,6-$Cl_3$ | | |
| 3. 17 | 3,4,5-$Cl_3$ | | |
| 3. 18 | 2,3,4,6-$Cl_4$ | | |
| 3. 19 | 2,3,5,6-$Cl_4$ | | |
| 3. 20 | 2,3,4,5,6-$Cl_5$ | | |
| 3. 21 | 2-Br | | |
| 3. 22 | 3-Br | | |
| 3. 23 | 4-Br | | |
| 3. 24 | 2,4-$Br_2$ | | |
| 3. 25 | 2,5-$Br_2$ | | |
| 3. 26 | 2,6-$Br_2$ | | |
| 3. 27 | 2,4,6-$Br_3$ | | |
| 3. 28 | 2,3,4,5,6-$Br_5$ | | |
| 3. 29 | 2-J | | |
| 3. 30 | 3-J | | |
| 3. 31 | 4-J | | |
| 3. 32 | 2,4-$J_2$ | | |
| 3. 33 | 2-Cl, 3-F | | |
| 3. 34 | 2-Cl, 4-F | | |
| 3. 35 | 2-Cl, 5-F | | |

Tabelle 3 (Fortsetzung)

| Nr. | $X_m$ | Fp(°C) | IR($cm^{-1}$) |
|---|---|---|---|
| 3. 36 | 2-Cl, 6-F | | |
| 3. 37 | 2-Cl, 3-Br | | |
| 3. 38 | 2-Cl, 4-Br | | |
| 3. 39 | 2-Cl, 5-Br | | |
| 3. 40 | 2-Cl, 6-Br | | |
| 3. 41 | 2-Br, 3-Cl | | |
| 3. 42 | 2-Br, 4-Cl | | |
| 3. 43 | 2-Br, 3-F | | |
| 3. 45 | 2-Br, 4-F | | |
| 3. 46 | 2-Br, 5-F | | |
| 3. 47 | 2-Br, 6-F | | |
| 3. 48 | 2-F, 3-Cl | | |
| 3. 49 | 2-F, 4-Cl | | |
| 3. 50 | 2-F, 5-Cl | | |
| 3. 51 | 3-Cl, 4-F | | |
| 3. 52 | 3-Cl, 5-F | | |
| 3. 53 | 3-Cl, 4-Br | | |
| 3. 54 | 3-Cl, 5-Br | | |
| 3. 55 | 3-F, 4-Cl | | |
| 3. 56 | 3-F, 4-Br | | |
| 3. 57 | 3-Br, 4-Cl | | |
| 3. 58 | 3-Br, 4-F | | |
| 3. 59 | 2,6-$Cl_2$, 4-Br | | |
| 3. 60 | 3-$CH_3$ | | |
| 3. 61 | 2,3-$(CH_3)_2$ | | |
| 3. 62 | 2,4-$(CH_3)_2$ | Öl | 3422, 2910, 1504, 1255, 1220, 1133, 1035, 759 |
| 3. 63 | 2,5-$(CH_3)_2$ | | |
| 3. 64 | 2,6-$(CH_3)_2$ | | |
| 3. 65 | 3,4-$(CH_3)_2$ | | |
| 3. 66 | 3,5-$(CH_3)_2$ | | |
| 3. 67 | 2,3,4-$(CH_3)_3$ | | |
| 3. 68 | 2,3,5-$(CH_3)_3$ | | |
| 3. 69 | 2,3,6-$(CH_3)_3$ | | |
| 3. 70 | 2,4,5-$(CH_3)_3$ | | |
| 3. 71 | 2,4,6-$(CH_3)_3$ | | |
| 3. 72 | 3,4,5-$(CH_3)_3$ | | |
| 3. 73 | 2,3,4,6-$(CH_3)_4$ | | |
| 3. 74 | 2,3,5,6-$(CH_3)_4$ | | |
| 3. 75 | 2,3,4,5,6-$(CH_3)_5$ | | |

Tabelle 3 (Fortsetzung)

| Nr. | $X_m$ | Fp(°C) | IR($cm^{-1}$) |
|---|---|---|---|
| 3. 76 | 2-$C_2H_5$ | | |
| 3. 77 | 3-$C_2H_5$ | | |
| 3. 78 | 4-$C_2H_5$ | | |
| 3. 79 | 2,4-$(C_2H_5)_2$ | | |
| 3. 80 | 2,6-$(C_2H_5)_2$ | | |
| 3. 81 | 3,5-$(C_2H_5)_2$ | | |
| 3. 82 | 2,4,6-$(C_2H_5)_3$ | | |
| 3. 83 | 2-n-$C_3H_7$ | | |
| 3. 84 | 3-n-$C_3H_7$ | | |
| 3. 85 | 4-n-$C_3H_7$ | | |
| 3. 86 | 2-i-$C_3H_7$ | | |
| 3. 87 | 3-i-$C_3H_7$ | | |
| 3. 88 | 4-i-$C_3H_7$ | | |
| 3. 89 | 2,4-(i-$C_3H_7$)$_2$ | | |
| 3. 90 | 2,6-(i-$C_3H_7$)$_2$ | | |
| 3. 91 | 3,5-(i-$C_3H_7$)$_2$ | | |
| 3. 92 | 2,4,6-(i-$C_3H_7$)$_3$ | | |
| 3. 93 | 2-s-$C_4H_9$ | | |
| 3. 94 | 3-s-$C_4H_9$ | | |
| 3. 95 | 4-s-$C_4H_9$ | | |
| 3. 96 | 2-t-$C_4H_9$ | | |
| 3. 97 | 3-t-$C_4H_9$ | | |
| 3. 98 | 2,3-(t-$C_4H_9$)$_2$ | | |
| 3. 99 | 2,4-(t-$C_4H_9$)$_2$ | | |
| 3.100 | 2,5-(t-$C_4H_9$)$_2$ | | |
| 3.101 | 2,6-(t-$C_4H_9$)$_2$ | | |
| 3.102 | 3,5-(t-$C_4H_9$)$_2$ | | |
| 3.103 | 2,4,6-(t-$C_4H_9$)$_3$ | | |
| 3.104 | 4-n-$C_9H_{19}$ | | |
| 3.105 | 4-n-$C_{12}H_{25}$ | | |
| 3.106 | 3-n-$C_{15}H_{31}$ | | |
| 3.107 | 4-(1,1,3,3-Tetramethylbutyl) | | |
| 3.108 | 4-(2,3,3-Trimethylpropyl) | | |
| 3.109 | 2-t-$C_4H_9$, 4-$CH_3$ | | |
| 3.110 | 2-t-$C_4H_9$, 5-$CH_3$ | | |
| 3.111 | 2,6-(t-$C_4H_9$)$_2$, 4-$CH_3$ | | |
| 3.112 | 2-$CH_3$, 4-t-$C_4H_9$ | | |
| 3.113 | 2-$CH_3$, 6-t-$C_4H_9$ | | |
| 3.114 | 2-$CH_3$, 4-i-$C_3H_7$ | | |

Tabelle 3 (Fortsetzung)

| Nr. | $X_m$ | Fp(°C) | IR($cm^{-1}$) |
|---|---|---|---|
| 3.115 | 2-$CH_3$, 5-i-$C_3H_7$ | | |
| 3.116 | 3-$CH_3$, 4-i-$C_3H_7$ | | |
| 3.117 | 2-i-$C_3H_7$, 5-$CH_3$ | | |
| 3.118 | 2,4-(t-$C_4H_9$)$_2$, 6-i-$C_3H_7$ | | |
| 3.119 | 2-$C_3H_5$ (= Allyl) | | |
| 3.120 | 3-$C_3H_5$ | | |
| 3.121 | 4-$C_3H_5$ | | |
| 3.122 | 2-$C_3H_5$, 6-$CH_3$ | | |
| 3.123 | 2-cyclo-$C_6H_{11}$ | | |
| 3.124 | 3-cyclo-$C_6H_{11}$ | | |
| 3.125 | 4-cyclo-$C_6H_{11}$ | | |
| 3.126 | 2,4-(cyclo-$C_6H_{11}$)$_2$, 6-$CH_3$ | | |
| 3.127 | 2-$CH_3$, 4-cyclo-$C_6H_{11}$ | | |
| 3.128 | 2-$CH_2C_6H_5$ | | |
| 3.129 | 3-$CH_2C_6H_5$ | | |
| 3.130 | 4-$CH_2C_6H_5$ | | |
| 3.131 | 2-$CH_2C_6H_5$, 4-$CH_3$ | | |
| 3.132 | 2-$CH_3$, 4-$CH_2C_6H_5$ | | |
| 3.133 | 2-$C_6H_5$ | | |
| 3.134 | 3-$C_6H_5$ | | |
| 3.135 | 4-$C_6H_5$ | | |
| 3.136 | 4-(2-i-$C_3H_7$-$C_6H_4$) | | |
| 3.137 | 4-$C_6H_5$, 2,6-($CH_3$)$_2$ | | |
| 3.138 | 2-Cl, 4-$C_6H_5$ | | |
| 3.139 | 2-Br, 4-$C_6H_5$ | | |
| 3.140 | 2-$C_6H_5$, 4-Cl | | |
| 3.141 | 2-$C_6H_5$, 4-Br | | |
| 3.142 | 2-$CH_2C_6H_5$, 4-Cl | | |
| 3.143 | 2-$CH_2C_6H_5$, 4-Br | | |
| 3.144 | 2-Cl, 4-$CH_2C_6H_5$ | | |
| 3.145 | 2-Br, 4-$CH_2C_6H_5$ | | |
| 3.146 | 2-cyclo-$C_6H_{11}$, 4-Cl | | |
| 3.147 | 2-cyclo-$C_6H_{11}$, 4-Br | | |
| 3.148 | 2-Cl, 4-cyclo-$C_6H_{11}$ | | |
| 3.149 | 2-Br, 4-cyclo-$C_6H_{11}$ | | |
| 3.150 | 3-$OCH_3$ | | |
| 3.151 | 2,4-($OCH_3$)$_2$ | | |
| 3.152 | 2-$CF_3$ | | |
| 3.153 | 3-$CF_3$ | | |

Tabelle 3 (Fortsetzung)

| Nr. | $X_m$ | Fp(°C) | IR(cm$^{-1}$) |
|---|---|---|---|
| 3.154 | 4-$CF_3$ | | |
| 3.155 | 2-$OCF_3$ | | |
| 3.156 | 3-$OCF_3$ | | |
| 3.157 | 4-$OCF_3$ | | |
| 3.158 | 3-$OCH_2CHF_2$ | | |
| 3.159 | 2-$NO_2$ | | |
| 3.160 | 3-$NO_2$ | | |
| 3.161 | 2-CN | | |
| 3.162 | 3-CN | | |
| 3.163 | 4-CN | | |
| 3.164 | 2-$CH_3$, 3-Cl | | |
| 3.165 | 2-$CH_3$, 5-Cl | | |
| 3.166 | 2-$CH_3$, 6-Cl | | |
| 3.167 | 2-$CH_3$, 3-F | | |
| 3.168 | 2-$CH_3$, 4-F | | |
| 3.169 | 2-$CH_3$, 5-F | | |
| 3.170 | 2-$CH_3$, 6-F | | |
| 3.171 | 2-$CH_3$, 3-Br | | |
| 3.172 | 2-$CH_3$, 4-Br | | |
| 3.173 | 2-$CH_3$, 5-Br | | |
| 3.174 | 2-$CH_3$, 6-Br | | |
| 3.175 | 2-Cl, 3-$CH_3$ | | |
| 3.176 | 2-Cl, 4-$CH_3$ | | |
| 3.177 | 2-Cl, 5-$CH_3$ | | |
| 3.178 | 2-F, 3-$CH_3$ | | |
| 3.179 | 2-F, 4-$CH_3$ | | |
| 3.180 | 2-F, 5-$CH_3$ | | |
| 3.181 | 2-Br, 3-$CH_3$ | | |
| 3.182 | 2-Br, 4-$CH_3$ | | |
| 3.183 | 3-$CH_3$, 4-Cl | | |
| 3.184 | 3-$CH_3$, 5-Cl | | |
| 3.185 | 2-Br, 5-$CH_3$ | | |
| 3.186 | 3-$CH_3$, 4-F | | |
| 3.187 | 3-$CH_3$, 5-F | | |
| 3.188 | 3-$CH_3$, 4-Br | | |
| 3.189 | 3-$CH_3$, 5-Br | | |
| 3.190 | 3-F, 4-$CH_3$ | | |
| 3.191 | 3-Cl, 4-$CH_3$ | | |
| 3.192 | 3-Br, 4-$CH_3$ | | |

Tabelle 3 (Fortsetzung)

| Nr. | $X_m$ | Fp($^oC$) | IR($cm^{-1}$) |
|---|---|---|---|
| 3.193 | 2-Cl, 4,5-$(CH_3)_2$ | | |
| 3.194 | 2-Br, 4,5-$(CH_3)_2$ | | |
| 3.195 | 2-Cl, 3,5-$(CH_3)_2$ | | |
| 3.196 | 2-Br, 3,5-$(CH_3)_2$ | | |
| 3.197 | 2,6-$Cl_2$, 4-$CH_3$ | | |
| 3.198 | 2,6-$F_2$, 4-$CH_3$ | | |
| 3.199 | 2,6-$Br_2$, 4-$CH_3$ | | |
| 3.200 | 2,4-$Cl_2$, 6-$CH_3$ | | |
| 3.201 | 2,4-$F_2$, 6-$CH_3$ | | |
| 3.202 | 2,4-$Br_2$, 6-$CH_3$ | | |
| 3.203 | 2,6-$(CH_3)_2$, 4-F | | |
| 3.204 | 2,6-$(CH_3)_2$, 4-Cl | | |
| 3.205 | 2,6-$(CH_3)_2$, 4-Br | | |
| 3.206 | 3,5-$(CH_3)_2$, 4-F | | |
| 3.207 | 3,5-$(CH_3)_2$, 4-Cl | | |
| 3.208 | 3,5-$(CH_3)_2$, 4-Br | | |
| 3.209 | 2,3,6-$(CH_3)_3$, 4-F | | |
| 3.210 | 2,3,6-$(CH_3)_3$, 4-Cl | | |
| 3.211 | 2,3,6-$(CH_3)_3$, 4-Br | | |
| 3.212 | 2,4-$(CH_3)_2$, 6-F | | |
| 3.213 | 2,4-$(CH_3)_2$, 6-Cl | | |
| 3.214 | 2,4-$(CH_3)_2$, 6-Br | | |
| 3.215 | 2-i-$C_3H_7$, 4-Cl, 5-$CH_3$ | | |
| 3.216 | 2-Cl, 4-$NO_2$ | | |
| 3.217 | 2-$NO_2$, 4-Cl | | |
| 3.218 | 2-$OCH_3$, 5-$NO_2$ | | |
| 3.219 | 2,4-$Cl_2$, 5-$NO_2$ | | |
| 3.220 | 2,4-$Cl_2$, 6-$NO_2$ | | |
| 3.221 | 2,6-$Cl_2$, 4-$NO_2$ | | |
| 3.222 | 2,6-$Br_2$, 4-$NO_2$ | | |
| 3.223 | 2,6-$J_2$, 4-$NO_2$ | | |
| 3.224 | 2-$CH_3$, 5-i-$C_3H_7$, 4-Cl | | |
| 3.225 | 2-$C_6H_5O$ | | |
| 3.226 | 3-$C_6H_5O$ | | |
| 3.227 | 4-$C_6H_5O$ | | |
| 3.228 | 3-t-$C_4H_9O$ | | |
| 3.229 | 4-t-$C_4H_9O$ | | |

Tabelle 3 (Fortsetzung)

| Nr. | $X_m$ | Fp($^{o}$C) | IR($cm^{-1}$) |
| --- | --- | --- | --- |
| 3.230 | *1) | | |
| 3.231 | *2) | | |

*1) 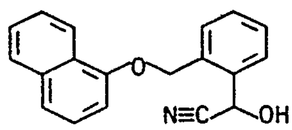

*2) 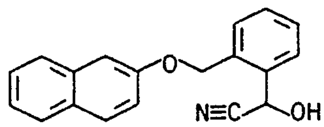

Tabelle 4

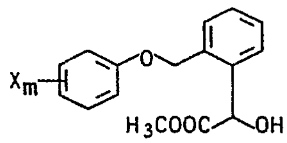

(III)

| Nr. | $X_m$ | Fp(°C) | IR($cm^{-1}$) |
|---|---|---|---|
| 4. 1 | 3-F | | |
| 4. 2 | 4-F | | |
| 4. 3 | 2,4-$F_2$ | | |
| 4. 4 | 2,4,6-$F_3$ | | |
| 4. 5 | 2,3,4,5,6-$F_5$ | | |
| 4. 6 | 2,3-$F_2$ | | |
| 4. 7 | 2,3-$Cl_2$ | | |
| 4. 8 | 2,5-$Cl_2$ | | |
| 4. 9 | 2,6-$Cl_2$ | | |
| 4. 10 | 3,4-$Cl_2$ | | |
| 4. 11 | 3,5-$Cl_2$ | | |
| 4. 12 | 2,3,4-$Cl_3$ | | |
| 4. 13 | 2,3,5-$Cl_3$ | | |
| 4. 14 | 2,3,6-$Cl_3$ | | |
| 4. 15 | 2,4,5-$Cl_3$ | | |
| 4. 16 | 2,4,6-$Cl_3$ | | |
| 4. 17 | 3,4,5-$Cl_3$ | | |
| 4. 18 | 2,3,4,6-$Cl_4$ | | |
| 4. 19 | 2,3,5,6-$Cl_4$ | | |
| 4. 20 | 2,3,4,5,6-$Cl_5$ | | |
| 4. 21 | 2-Br | | |
| 4. 22 | 3-Br | | |
| 4. 23 | 4-Br | | |
| 4. 24 | 2,4-$Br_2$ | | |
| 4. 25 | 2,5-$Br_2$ | | |
| 4. 26 | 2,6-$Br_2$ | | |
| 4. 27 | 2,4,6-$Br_3$ | | |
| 4. 28 | 2,3,4,5,6-$Br_5$ | | |
| 4. 29 | 2-J | | |
| 4. 30 | 3-J | | |
| 4. 31 | 4-J | | |
| 4. 32 | 2,4-$J_2$ | | |
| 4. 33 | 2-Cl, 3-F | | |
| 4. 34 | 2-Cl, 4-F | | |

Tabelle 4 (Fortsetzung)

| Nr. | $X_m$ | Fp(°C) | IR($cm^{-1}$) |
|---|---|---|---|
| 4. 35 | 2-Cl, 5-F | | |
| 4. 36 | 2-Cl, 6-F | | |
| 4. 37 | 2-Cl, 3-Br | | |
| 4. 38 | 2-Cl, 4-Br | | |
| 4. 39 | 2-Cl, 5-Br | | |
| 4. 40 | 2-Cl, 6-Br | | |
| 4. 41 | 2-Br, 3-Cl | | |
| 4. 42 | 2-Br, 4-Cl | | |
| 4. 43 | 2-Br, 3-F | | |
| 4. 45 | 2-Br, 4-F | | |
| 4. 46 | 2-Br, 5-F | | |
| 4. 47 | 2-Br, 6-F | | |
| 4. 48 | 2-F, 3-Cl | | |
| 4. 49 | 2-F, 4-Cl | | |
| 4. 50 | 2-F, 5-Cl | | |
| 4. 51 | 3-Cl, 4-F | | |
| 4. 52 | 3-Cl, 5-F | | |
| 4. 53 | 3-Cl, 4-Br | | |
| 4. 54 | 3-Cl, 5-Br | | |
| 4. 55 | 3-F, 4-Cl | | |
| 4. 56 | 3-F, 4-Br | | |
| 4. 57 | 3-Br, 4-Cl | | |
| 4. 58 | 3-Br, 4-F | | |
| 4. 59 | 2,6-$Cl_2$, 4-Br | | |
| 4. 60 | 3-$CH_3$ | | |
| 4. 61 | 2,3-$(CH_3)_2$ | | |
| 4. 62 | 2,4-$(CH_3)_2$ | öl | 3460,2915,1739,1503,1254,1221 |
| 4. 63 | 2,5-$(CH_3)_2$ | | |
| 4. 64 | 2,6-$(CH_3)_2$ | | |
| 4. 65 | 3,4-$(CH_3)_2$ | | |
| 4. 66 | 3,5-$(CH_3)_2$ | | |
| 4. 67 | 2,3,4-$(CH_3)_3$ | | |
| 4. 68 | 2,3,5-$(CH_3)_3$ | | |
| 4. 69 | 2,3,6-$(CH_3)_3$ | | |
| 4. 70 | 2,4,5-$(CH_3)_3$ | | |
| 4. 71 | 2,4,6-$(CH_3)_3$ | | |
| 4. 72 | 3,4,5-$(CH_3)_3$ | | |
| 4. 73 | 2,3,4,6-$(CH_3)_4$ | | |
| 4. 74 | 2,3,5,6-$(CH_3)_4$ | | |

Tabelle 4 (Fortsetzung)

| Nr. | $X_m$ | Fp(°C) | IR(cm⁻¹) |
|---|---|---|---|
| 4. 75 | 2,3,4,5,6-$(CH_3)_5$ | | |
| 4. 76 | 2-$C_2H_5$ | | |
| 4. 77 | 3-$C_2H_5$ | | |
| 4. 78 | 4-$C_2H_5$ | | |
| 4. 79 | 2,4-$(C_2H_5)_2$ | | |
| 4. 80 | 2,6-$(C_2H_5)_2$ | | |
| 4. 81 | 3,5-$(C_2H_5)_2$ | | |
| 4. 82 | 2,4,6-$(C_2H_5)_3$ | | |
| 4. 83 | 2-n-$C_3H_7$ | | |
| 4. 84 | 3-n-$C_3H_7$ | | |
| 4. 85 | 4-n-$C_3H_7$ | | |
| 4. 86 | 2-i-$C_3H_7$ | | |
| 4. 87 | 3-i-$C_3H_7$ | | |
| 4. 88 | 4-i-$C_3H_7$ | | |
| 4. 89 | 2,4-(i-$C_3H_7)_2$ | | |
| 4. 90 | 2,6-(i-$C_3H_7)_2$ | | |
| 4. 91 | 3,5-(i-$C_3H_7)_2$ | | |
| 4. 92 | 2,4,6-(i-$C_3H_7)_3$ | | |
| 4. 93 | 2-s-$C_4H_9$ | | |
| 4. 94 | 3-s-$C_4H_9$ | | |
| 4. 95 | 4-s-$C_4H_9$ | | |
| 4. 96 | 2-t-$C_4H_9$ | | |
| 4. 97 | 3-t-$C_4H_9$ | | |
| 4. 98 | 2,3-(t-$C_4H_9)_2$ | | |
| 4. 99 | 2,4-(t-$C_4H_9)_2$ | | |
| 4.100 | 2,5-(t-$C_4H_9)_2$ | | |
| 4.101 | 2,6-(t-$C_4H_9)_2$ | | |
| 4.102 | 3,5-(t-$C_4H_9)_2$ | | |
| 4.103 | 2,4,6-(t-$C_4H_9)_3$ | | |
| 4.104 | 4-n-$C_9H_{19}$ | | |
| 4.105 | 4-n-$C_{12}H_{25}$ | | |
| 4.106 | 3-n-$C_{15}H_{31}$ | | |
| 4.107 | 4-(1,1,3,3-Tetramethylbutyl) | | |
| 4.108 | 4-(2,3,3-Trimethylpropyl) | | |
| 4.109 | 2-t-$C_4H_9$, 4-$CH_3$ | | |
| 4.110 | 2-t-$C_4H_9$, 5-$CH_3$ | | |
| 4.111 | 2,6-(t-$C_4H_9)_2$, 4-$CH_3$ | | |
| 4.112 | 2-$CH_3$, 4-t-$C_4H_9$ | | |
| 4.113 | 2-$CH_3$, 6-t-$C_4H_9$ | | |

Tabelle 4 (Fortsetzung)

| Nr. | $X_m$ | Fp(°C) | IR($cm^{-1}$) |
|---|---|---|---|
| 4.114 | 2-$CH_3$, 4-i-$C_3H_7$ | | |
| 4.115 | 2-$CH_3$, 5-i-$C_3H_7$ | | |
| 4.116 | 3-$CH_3$, 4-i-$C_3H_7$ | | |
| 4.117 | 2-i-$C_3H_7$, 5-$CH_3$ | | |
| 4.118 | 2,4-(t-$C_4H_9$)$_2$, 6-i-$C_3H_7$ | | |
| 4.119 | 2-$C_3H_5$ (= Allyl) | | |
| 4.120 | 3-$C_3H_5$ | | |
| 4.121 | 4-$C_3H_5$ | | |
| 4.122 | 2-$C_3H_5$, 6-$CH_3$ | | |
| 4.123 | 2-cyclo-$C_6H_{11}$ | | |
| 4.124 | 3-cyclo-$C_6H_{11}$ | | |
| 4.125 | 4-cyclo-$C_6H_{11}$ | | |
| 4.126 | 2,4-(cyclo-$C_6H_{11}$)$_2$, 6-$CH_3$ | | |
| 4.127 | 2-$CH_3$, 4-cyclo-$C_6H_{11}$ | | |
| 4.128 | 2-$CH_2C_6H_5$ | | |
| 4.129 | 3-$CH_2C_6H_5$ | | |
| 4.130 | 4-$CH_2C_6H_5$ | | |
| 4.131 | 2-$CH_2C_6H_5$, 4-$CH_3$ | | |
| 4.132 | 2-$CH_3$, 4-$CH_2C_6H_5$ | | |
| 4.133 | 2-$C_6H_5$ | | |
| 4.134 | 3-$C_6H_5$ | | |
| 4.135 | 4-$C_6H_5$ | | |
| 4.136 | 4-(2-i-$C_3H_7$-$C_6H_4$) | | |
| 4.137 | 4-$C_6H_5$, 2,6-($CH_3$)$_2$ | | |
| 4.138 | 2-Cl, 4-$C_6H_5$ | | |
| 4.139 | 2-Br, 4-$C_6H_5$ | | |
| 4.140 | 2-$C_6H_5$, 4-Cl | | |
| 4.141 | 2-$C_6H_5$, 4-Br | | |
| 4.142 | 2-$CH_2C_6H_5$, 4-Cl | | |
| 4.143 | 2-$CH_2C_6H_5$, 4-Br | | |
| 4.144 | 2-Cl, 4-$CH_2C_6H_5$ | | |
| 4.145 | 2-Br, 4-$CH_2C_6H_5$ | | |
| 4.146 | 2-cyclo-$C_6H_{11}$, 4-Cl | | |
| 4.147 | 2-cyclo-$C_6H_{11}$, 4-Br | | |
| 4.148 | 2-Cl, 4-cyclo-$C_6H_{11}$ | | |
| 4.149 | 2-Br, 4-cyclo-$C_6H_{11}$ | | |
| 4.150 | 3-$OCH_3$ | | |
| 4.151 | 2,4-($OCH_3$)$_2$ | | |
| 4.152 | 2-$CF_3$ | | |

Tabelle 4 (Fortsetzung)

| Nr. | $X_m$ | Fp(°C) | IR($cm^{-1}$) |
|---|---|---|---|
| 4.153 | 3-$CF_3$ | | |
| 4.154 | 4-$CF_3$ | | |
| 4.155 | 2-$OCF_3$ | | |
| 4.156 | 3-$OCF_3$ | | |
| 4.157 | 4-$OCF_3$ | | |
| 4.158 | 3-$OCH_2CHF_2$ | | |
| 4.159 | 2-$NO_2$ | | |
| 4.160 | 3-$NO_2$ | | |
| 4.161 | 2-CN | | |
| 4.162 | 3-CN | | |
| 4.163 | 4-CN | | |
| 4.164 | 2-$CH_3$, 3-Cl | | |
| 4.165 | 2-$CH_3$, 5-Cl | | |
| 4.166 | 2-$CH_3$, 6-Cl | | |
| 4.167 | 2-$CH_3$, 3-F | | |
| 4.168 | 2-$CH_3$, 4-F | | |
| 4.169 | 2-$CH_3$, 5-F | | |
| 4.170 | 2-$CH_3$, 6-F | | |
| 4.171 | 2-$CH_3$, 3-Br | | |
| 4.172 | 2-$CH_3$, 4-Br | | |
| 4.173 | 2-$CH_3$, 5-Br | | |
| 4.174 | 2-$CH_3$, 6-Br | | |
| 4.175 | 2-Cl, 3-$CH_3$ | | |
| 4.176 | 2-Cl, 4-$CH_3$ | | |
| 4.177 | 2-Cl, 5-$CH_3$ | | |
| 4.178 | 2-F, 3-$CH_3$ | | |
| 4.179 | 2-F, 4-$CH_3$ | | |
| 4.180 | 2-F, 5-$CH_3$ | | |
| 4.181 | 2-Br, 3-$CH_3$ | | |
| 4.182 | 2-Br, 4-$CH_3$ | | |
| 4.183 | 3-$CH_3$, 4-Cl | | |
| 4.184 | 3-$CH_3$, 5-Cl | | |
| 4.185 | 2-Br, 5-$CH_3$ | | |
| 4.186 | 3-$CH_3$, 4-F | | |
| 4.187 | 3-$CH_3$, 5-F | | |
| 4.188 | 3-$CH_3$, 4-Br | | |
| 4.189 | 3-$CH_3$, 5-Br | | |
| 4.190 | 3-F, 4-$CH_3$ | | |
| 4.191 | 3-Cl, 4-$CH_3$ | | |

Tabelle 4 (Fortsetzung)

| Nr. | $X_m$ | Fp(°C) | IR($cm^{-1}$) |
|---|---|---|---|
| 4.192 | 3-Br, 4-$CH_3$ | | |
| 4.193 | 2-Cl, 4,5-$(CH_3)_2$ | | |
| 4.194 | 2-Br, 4,5-$(CH_3)_2$ | | |
| 4.195 | 2-Cl, 3,5-$(CH_3)_2$ | | |
| 4.196 | 2-Br, 3,5-$(CH_3)_2$ | | |
| 4.197 | 2,6-$Cl_2$, 4-$CH_3$ | | |
| 4.198 | 2,6-$F_2$, 4-$CH_3$ | | |
| 4.199 | 2,6-$Br_2$, 4-$CH_3$ | | |
| 4.200 | 2,4-$Cl_2$, 6-$CH_3$ | | |
| 4.201 | 2,4-$F_2$, 6-$CH_3$ | | |
| 4.202 | 2,4-$Br_2$, 6-$CH_3$ | | |
| 4.203 | 2,6-$(CH_3)_2$, 4-F | | |
| 4.204 | 2,6-$(CH_3)_2$, 4-Cl | | |
| 4.205 | 2,6-$(CH_3)_2$, 4-Br | | |
| 4.206 | 3,5-$(CH_3)_2$, 4-F | | |
| 4.207 | 3,5-$(CH_3)_2$, 4-Cl | | |
| 4.208 | 3,5-$(CH_3)_2$, 4-Br | | |
| 4.209 | 2,3,6-$(CH_3)_3$, 4-F | | |
| 4.210 | 2,3,6-$(CH_3)_3$, 4-Cl | | |
| 4.211 | 2,3,6-$(CH_3)_3$, 4-Br | | |
| 4.212 | 2,4-$(CH_3)_2$, 6-F | | |
| 4.213 | 2,4-$(CH_3)_2$, 6-Cl | | |
| 4.214 | 2,4-$(CH_3)_2$, 6-Br | | |
| 4.215 | 2-i-$C_3H_7$, 4-Cl, 5-$CH_3$ | | |
| 4.216 | 2-Cl, 4-$NO_2$ | | |
| 4.217 | 2-$NO_2$, 4-Cl | | |
| 4.218 | 2-$OCH_3$, 5-$NO_2$ | | |
| 4.219 | 2,4-$Cl_2$, 5-$NO_2$ | | |
| 4.220 | 2,4-$Cl_2$, 6-$NO_2$ | | |
| 4.221 | 2,6-$Cl_2$, 4-$NO_2$ | | |
| 4.222 | 2,6-$Br_2$, 4-$NO_2$ | | |
| 4.223 | 2,6-$J_2$, 4-$NO_2$ | | |
| 4.224 | 2-$CH_3$, 5-i-$C_3H_7$, 4-Cl | | |
| 4.225 | 2-$C_6H_5O$ | | |
| 4.226 | 3-$C_6H_5O$ | | |
| 4.227 | 4-$C_6H_5O$ | | |
| 4.228 | 3-t-$C_4H_9O$ | | |
| 4.229 | 4-t-$C_4H_9O$ | | |

Tabelle 4 (Fortsetzung)

| Nr. | $X_m$ | Fp(°C) | IR($cm^{-1}$) |
|---|---|---|---|
| 4.230 | *1) | | |
| 4.231 | *2) | | |

*1) 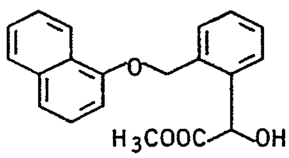

*2) 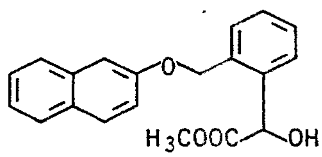

Tabelle 5

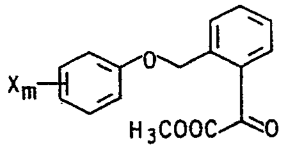

(II)

| Nr. | $X_m$ | Fp(°C) | IR($cm^{-1}$) |
|---|---|---|---|
| 5. 1 | 3-F | | |
| 5. 2 | 4-F | | |
| 5. 3 | 2,4-$F_2$ | | |
| 5. 4 | 2,4,6-$F_3$ | | |
| 5. 5 | 2,3,4,5,6-$F_5$ | | |
| 5. 6 | 2,3-$F_2$ | | |
| 5. 7 | 2,3-$Cl_2$ | | |
| 5. 8 | 2,5-$Cl_2$ | | |
| 5. 9 | 2,6-$Cl_2$ | | |
| 5. 10 | 3,4-$Cl_2$ | | |
| 5. 11 | 3,5-$Cl_2$ | | |
| 5. 12 | 2,3,4-$Cl_3$ | | |
| 5. 13 | 2,3,5-$Cl_3$ | | |
| 5. 14 | 2,3,6-$Cl_3$ | | |
| 5. 15 | 2,4,5-$Cl_3$ | | |
| 5. 16 | 2,4,6-$Cl_3$ | | |
| 5. 17 | 3,4,5-$Cl_3$ | | |
| 5. 18 | 2,3,4,6-$Cl_4$ | | |
| 5. 19 | 2,3,5,6-$Cl_4$ | | |
| 5. 20 | 2,3,4,5,6-$Cl_5$ | | |
| 5. 21 | 2-Br | | |
| 5. 22 | 3-Br | | |
| 5. 23 | 4-Br | | |
| 5. 24 | 2,4-$Br_2$ | | |
| 5. 25 | 2,5-$Br_2$ | | |
| 5. 26 | 2,6-$Br_2$ | | |
| 5. 27 | 2,4,6-$Br_3$ | | |
| 5. 28 | 2,3,4,5,6-$Br_5$ | | |
| 5. 29 | 2-J | | |
| 5. 30 | 3-J | | |
| 5. 31 | 4-J | | |
| 5. 32 | 2,4-$J_2$ | | |
| 5. 33 | 2-Cl, 3-F | | |
| 5. 34 | 2-Cl, 4-F | | |

Tabelle 5 (Fortsetzung)

| Nr. | $X_m$ | Fp(°C) | IR($cm^{-1}$) |
|---|---|---|---|
| 5. 35 | 2-Cl, 5-F | | |
| 5. 36 | 2-Cl, 6-F | | |
| 5. 37 | 2-Cl, 3-Br | | |
| 5. 38 | 2-Cl, 4-Br | | |
| 5. 39 | 2-Cl, 5-Br | | |
| 5. 40 | 2-Cl, 6-Br | | |
| 5. 41 | 2-Br, 3-Cl | | |
| 5. 42 | 2-Br, 4-Cl | | |
| 5. 43 | 2-Br, 3-F | | |
| 5. 45 | 2-Br, 4-F | | |
| 5. 46 | 2-Br, 5-F | | |
| 5. 47 | 2-Br, 6-F | | |
| 5. 48 | 2-F, 3-Cl | | |
| 5. 49 | 2-F, 4-Cl | | |
| 5. 50 | 2-F, 5-Cl | | |
| 5. 51 | 3-Cl, 4-F | | |
| 5. 52 | 3-Cl, 5-F | | |
| 5. 53 | 3-Cl, 4-Br | | |
| 5. 54 | 3-Cl, 5-Br | | |
| 5. 55 | 3-F, 4-Cl | | |
| 5. 56 | 3-F, 4-Br | | |
| 5. 57 | 3-Br, 4-Cl | | |
| 5. 58 | 3-Br, 4-F | | |
| 5. 59 | 2,6-$Cl_2$, 4-Br | | |
| 5. 60 | 3-$CH_3$ | | 1732,1699,1601,1258,1204,1156, 760 |
| 5. 61 | 2,3-$(CH_3)_2$ | | |
| 5. 62 | 2,4-$(CH_3)_2$ | | 1734,1678,1505,1258,1207,1137,1007,796 |
| 5. 63 | 2,5-$(CH_3)_2$ | | |
| 5. 64 | 2,6-$(CH_3)_2$ | | |
| 5. 65 | 3,4-$(CH_3)_2$ | | |
| 5. 66 | 3,5-$(CH_3)_2$ | | |
| 5. 67 | 2,3,4-$(CH_3)_3$ | | |
| 5. 68 | 2,3,5-$(CH_3)_3$ | | |
| 5. 69 | 2,3,6-$(CH_3)_3$ | | |
| 5. 70 | 2,4,5-$(CH_3)_3$ | | |
| 5. 71 | 2,4,6-$(CH_3)_3$ | | |
| 5. 72 | 3,4,5-$(CH_3)_3$ | | |
| 5. 73 | 2,3,4,6-$(CH_3)_4$ | | |
| 5. 74 | 2,3,5,6-$(CH_3)_4$ | | |

Tabelle 5 (Fortsetzung)

| Nr. | $X_m$ | Fp(°C) | IR($cm^{-1}$) |
|---|---|---|---|
| 5. 75 | 2,3,4,5,6-$(CH_3)_5$ | | |
| 5. 76 | 2-$C_2H_5$ | | |
| 5. 77 | 3-$C_2H_5$ | | |
| 5. 78 | 4-$C_2H_5$ | | |
| 5. 79 | 2,4-$(C_2H_5)_2$ | | |
| 5. 80 | 2,6-$(C_2H_5)_2$ | | |
| 5. 81 | 3,5-$(C_2H_5)_2$ | | |
| 5. 82 | 2,4,6-$(C_2H_5)_3$ | | |
| 5. 83 | 2-n-$C_3H_7$ | | |
| 5. 84 | 3-n-$C_3H_7$ | | |
| 5. 85 | 4-n-$C_3H_7$ | | |
| 5. 86 | 2-i-$C_3H_7$ | | |
| 5. 87 | 3-i-$C_3H_7$ | | |
| 5. 88 | 4-i-$C_3H_7$ | | |
| 5. 89 | 2,4-(i-$C_3H_7)_2$ | | |
| 5. 90 | 2,6-(i-$C_3H_7)_2$ | | |
| 5. 91 | 3,5-(i-$C_3H_7)_2$ | | |
| 5. 92 | 2,4,6-(i-$C_3H_7)_3$ | | |
| 5. 93 | 2-s-$C_4H_9$ | | |
| 5. 94 | 3-s-$C_4H_9$ | | |
| 5. 95 | 4-s-$C_4H_9$ | | |
| 5. 96 | 2-t-$C_4H_9$ | | |
| 5. 97 | 3-t-$C_4H_9$ | | |
| 5. 98 | 2,3-(t-$C_4H_9)_2$ | | |
| 5. 99 | 2,4-(t-$C_4H_9)_2$ | | |
| 5.100 | 2,5-(t-$C_4H_9)_2$ | | |
| 5.101 | 2,6-(t-$C_4H_9)_2$ | | |
| 5.102 | 3,5-(t-$C_4H_9)_2$ | | |
| 5.103 | 2,4,6-(t-$C_4H_9)_3$ | | |
| 5.104 | 4-n-$C_9H_{19}$ | | |
| 5.105 | 4-n-$C_{12}H_{25}$ | | |
| 5.106 | 3-n-$C_{15}H_{31}$ | | |
| 5.107 | 4-(1,1,3,3-Tetramethylbutyl) | | |
| 5.108 | 4-(2,3,3-Trimethylpropyl) | | |
| 5.109 | 2-t-$C_4H_9$, 4-$CH_3$ | | |
| 5.110 | 2-t-$C_4H_9$, 5-$CH_3$ | | |
| 5.111 | 2,6-(t-$C_4H_9)_2$, 4-$CH_3$ | | |
| 5.112 | 2-$CH_3$, 4-t-$C_4H_9$ | | |
| 5.113 | 2-$CH_3$, 6-t-$C_4H_9$ | | |

Tabelle 5 (Fortsetzung)

| Nr. | $X_m$ | Fp(°C) | IR($cm^{-1}$) |
|---|---|---|---|
| 5.114 | 2-$CH_3$, 4-i-$C_3H_7$ | | |
| 5.115 | 2-$CH_3$, 5-i-$C_3H_7$ | | |
| 5.116 | 3-$CH_3$, 4-i-$C_3H_7$ | | |
| 5.117 | 2-i-$C_3H_7$, 5-$CH_3$ | | |
| 5.118 | 2,4-(t-$C_4H_9$)$_2$, 6-i-$C_3H_7$ | | |
| 5.119 | 2-$C_3H_5$ (= Allyl) | | |
| 5.120 | 3-$C_3H_5$ | | |
| 5.121 | 4-$C_3H_5$ | | |
| 5.122 | 2-$C_3H_5$, 6-$CH_3$ | | |
| 5.123 | 2-cyclo-$C_6H_{11}$ | | |
| 5.124 | 3-cyclo-$C_6H_{11}$ | | |
| 5.125 | 4-cyclo-$C_6H_{11}$ | | |
| 5.126 | 2,4-(cyclo-$C_6H_{11}$)$_2$, 6-$CH_3$ | | |
| 5.127 | 2-$CH_3$, 4-cyclo-$C_6H_{11}$ | | |
| 5.128 | 2-$CH_2C_6H_5$ | | |
| 5.129 | 3-$CH_2C_6H_5$ | | |
| 5.130 | 4-$CH_2C_6H_5$ | | |
| 5.131 | 2-$CH_2C_6H_5$, 4-$CH_3$ | | |
| 5.132 | 2-$CH_3$, 4-$CH_2C_6H_5$ | | |
| 5.133 | 2-$C_6H_5$ | | |
| 5.134 | 3-$C_6H_5$ | | |
| 5.135 | 4-$C_6H_5$ | | |
| 5.136 | 4-(2-i-$C_3H_7$-$C_6H_4$) | | |
| 5.137 | 4-$C_6H_5$, 2,6-($CH_3$)$_2$ | | |
| 5.138 | 2-Cl, 4-$C_6H_5$ | | |
| 5.139 | 2-Br, 4-$C_6H_5$ | | |
| 5.140 | 2-$C_6H_5$, 4-Cl | | |
| 5.141 | 2-$C_6H_5$, 4-Br | | |
| 5.142 | 2-$CH_2C_6H_5$, 4-Cl | | |
| 5.143 | 2-$CH_2C_6H_5$, 4-Br | | |
| 5.144 | 2-Cl, 4-$CH_2C_6H_5$ | | |
| 5.145 | 2-Br, 4-$CH_2C_6H_5$ | | |
| 5.146 | 2-cyclo-$C_6H_{11}$, 4-Cl | | |
| 5.147 | 2-cyclo-$C_6H_{11}$, 4-Br | | |
| 5.148 | 2-Cl, 4-cyclo-$C_6H_{11}$ | | |
| 5.149 | 2-Br, 4-cyclo-$C_6H_{11}$ | | |
| 5.150 | 3-$OCH_3$ | Öl | 1742,1670,1597,1276,1011, 755 |
| 5.151 | 2,4-($OCH_3$)$_2$ | | |
| 5.152 | 2-$CF_3$ | | |

Tabelle 5 (Fortsetzung)

| Nr. | $X_m$ | Fp(°C) | IR($cm^{-1}$) |
|---|---|---|---|
| 5.153 | 3-$CF_3$ | | |
| 5.154 | 4-$CF_3$ | | |
| 5.155 | 2-$OCF_3$ | | |
| 5.156 | 3-$OCF_3$ | | |
| 5.157 | 4-$OCF_3$ | | |
| 5.158 | 3-$OCH_2CHF_2$ | | |
| 5.159 | 2-$NO_2$ | | |
| 5.160 | 3-$NO_2$ | | |
| 5.161 | 2-CN | | |
| 5.162 | 3-CN | | |
| 5.163 | 4-CN | | |
| 5.164 | 2-$CH_3$, 3-Cl | | |
| 5.165 | 2-$CH_3$, 5-Cl | | |
| 5.166 | 2-$CH_3$, 6-Cl | | |
| 5.167 | 2-$CH_3$, 3-F | | |
| 5.168 | 2-$CH_3$, 4-F | | |
| 5.169 | 2-$CH_3$, 5-F | | |
| 5.170 | 2-$CH_3$, 6-F | | |
| 5.171 | 2-$CH_3$, 3-Br | | |
| 5.172 | 2-$CH_3$, 4-Br | | |
| 5.173 | 2-$CH_3$, 5-Br | | |
| 5.174 | 2-$CH_3$, 6-Br | | |
| 5.175 | 2-Cl, 3-$CH_3$ | | |
| 5.176 | 2-Cl, 4-$CH_3$ | | |
| 5.177 | 2-Cl, 5-$CH_3$ | | |
| 5.178 | 2-F, 3-$CH_3$ | | |
| 5.179 | 2-F, 4-$CH_3$ | | |
| 5.180 | 2-F, 5-$CH_3$ | | |
| 5.181 | 2-Br, 3-$CH_3$ | | |
| 5.182 | 2-Br, 4-$CH_3$ | | |
| 5.183 | 3-$CH_3$, 4-Cl | | |
| 5.184 | 3-$CH_3$, 5-Cl | | |
| 5.185 | 2-Br, 5-$CH_3$ | | |
| 5.186 | 3-$CH_3$, 4-F | | |
| 5.187 | 3-$CH_3$, 5-F | | |
| 5.188 | 3-$CH_3$, 4-Br | | |
| 5.189 | 3-$CH_3$, 5-Br | | |
| 5.190 | 3-F, 4-$CH_3$ | | |
| 5.191 | 3-Cl, 4-$CH_3$ | | |

Tabelle 5 (Fortsetzung)

| Nr. | $X_m$ | Fp(°C) | IR($cm^{-1}$) |
|---|---|---|---|
| 5.192 | 3-Br, 4-$CH_3$ | | |
| 5.193 | 2-Cl, 4,5-$(CH_3)_2$ | | |
| 5.194 | 2-Br, 4,5-$(CH_3)_2$ | | |
| 5.195 | 2-Cl, 3,5-$(CH_3)_2$ | | |
| 5.196 | 2-Br, 3,5-$(CH_3)_2$ | | |
| 5.197 | 2,6-$Cl_2$, 4-$CH_3$ | | |
| 5.198 | 2,6-$F_2$, 4-$CH_3$ | | |
| 5.199 | 2,6-$Br_2$, 4-$CH_3$ | | |
| 5.200 | 2,4-$Cl_2$, 6-$CH_3$ | | |
| 5.201 | 2,4-$F_2$, 6-$CH_3$ | | |
| 5.202 | 2,4-$Br_2$, 6-$CH_3$ | | |
| 5.203 | 2,6-$(CH_3)_2$, 4-F | | |
| 5.204 | 2,6-$(CH_3)_2$, 4-Cl | | |
| 5.205 | 2,6-$(CH_3)_2$, 4-Br | | |
| 5.206 | 3,5-$(CH_3)_2$, 4-F | | |
| 5.207 | 3,5-$(CH_3)_2$, 4-Cl | | |
| 5.208 | 3,5-$(CH_3)_2$, 4-Br | | |
| 5.209 | 2,3,6-$(CH_3)_3$, 4-F | | |
| 5.210 | 2,3,6-$(CH_3)_3$, 4-Cl | | |
| 5.211 | 2,3,6-$(CH_3)_3$, 4-Br | | |
| 5.212 | 2,4-$(CH_3)_2$, 6-F | | |
| 5.213 | 2,4-$(CH_3)_2$, 6-Cl | | |
| 5.214 | 2,4-$(CH_3)_2$, 6-Br | | |
| 5.215 | 2-i-$C_3H_7$, 4-Cl, 5-$CH_3$ | | |
| 5.216 | 2-Cl, 4-$NO_2$ | | |
| 5.217 | 2-$NO_2$, 4-Cl | | |
| 5.218 | 2-$OCH_3$, 5-$NO_2$ | | |
| 5.219 | 2,4-$Cl_2$, 5-$NO_2$ | | |
| 5.220 | 2,4-$Cl_2$, 6-$NO_2$ | | |
| 5.221 | 2,6-$Cl_2$, 4-$NO_2$ | | |
| 5.222 | 2,6-$Br_2$, 4-$NO_2$ | | |
| 5.223 | 2,6-$J_2$, 4-$NO_2$ | | |
| 5.224 | 2-$CH_3$, 5-i-$C_3H_7$, 4-Cl | | |
| 5.225 | 2-$C_6H_5O$ | | |
| 5.226 | 3-$C_6H_5O$ | | |
| 5.227 | 4-$C_6H_5O$ | | |
| 5.228 | 3-t-$C_4H_9O$ | | |
| 5.229 | 4-t-$C_4H_9O$ | | |

Tabelle 5 (Fortsetzung)

| Nr. | $X_m$ | Fp(°C) | IR(cm$^{-1}$) |
|---|---|---|---|
| 5.230 | *1) | | |
| 5.231 | *2) | | |

*1) 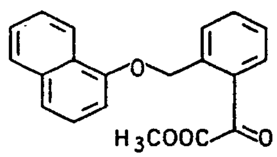

*2) 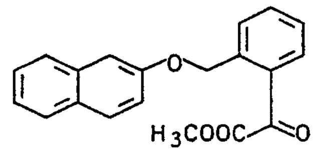

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle Soja, Kaffee, Zuckerrohr, Obst und Zierpflanzen im Gartenbau Weinbau sowie Gemüse - wie Gurken Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es wurden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungs zwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung

von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz und Holzschutz eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g, je Kilogramm Saatgut verwendet.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1.1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1.2 werden in einer Mischung gelöst, die aus 80 Gew.-Teilen Xylol, 10 Gew.-Teilen des Anlagerungsproduktes von 8 bis 10 Mol Ethylenoxid an 1 Mol Ösäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1.3 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1.7 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1.10 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1.21 werden mit 97 Gew.-Teilen feinteiligem Kaolin innig vermischt. Man erhält auf diese Weise ein Stäubemittel, das 3 Gew.% des Wirkstoffs enthält.

VII 30 Gew.-Teile der Verbindung Nr. 1.34 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1.38 werden mit 10 Gew.-Teilen Natriumsalz eines Phensolsulfonsäure-harnstoff-formaldehyd-Konden sates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1.153 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 2-Phenoxymethylphenylglyoxylsäuremethylester-O-methyloxim (A) - bekannt aus EP 253 213 - benutzt.

Anwendungsbeispiel

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt.

Anschließend wurden die Pflanzen im Gewächshaus bei 20 bis 22 °C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 1.1, 1.2, 1.3, 1.7, 1.10, 1.21, 1.34, 1.38, 1.45, 1.60, 1.61, 1.62, 1.72, 1.97, 1.115, 1.135, 1.138, 1.153 und 1.157 bei der Anwendung als 0,05 %ige (Gew.%) Spritzbrühe eine bessere fungizide Wirkung zeigen (97 %) als der bekannte Vergleichswirkstoff A (55 %).

**Ansprüche**

1. Oximether der Formel I

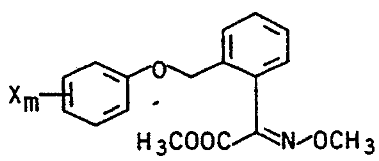

I

in der

X (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,

mit Ausnahme der Verbindungen, bei denen

$X_m$

2-Fluor-, 2-Chlor-, 3-Chlor-, 4-Chlor-, 2,4-Dichlor-, 2-Methyl-4-Chlor-, 2-Methyl-, 4-Methyl- 4-tert.-Butyl-, 2-Methoxy-, 4-Methoxy-, 2-Trifluormethyl- oder 4-Nitro bedeutet.

2. Verfahren zur Bekämpfung von Pilzen dadurch gekennzeichnet, daß man die Pilze oder die vom Pilzbefall bedrohten Materialien, Pflanzen, Saatgüter oder den Erdboden mit einer fungizid wirksamen Menge einer Verbindung der Formel I behandelt

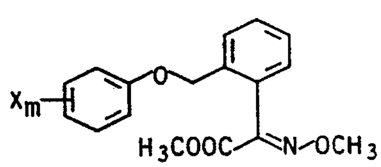

I

in der

X (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,

mit Ausnahme der Verbindungen, bei denen

$X_m$

2-Fluor-, 2-Chlor-, 3-Chlor-, 4-Chlor-, 2,4-Dichlor-, 2-Methyl-4-Chlor-, 2-Methyl-, 4-Methyl-, 4-tert.-Butyl-, 2-Methoxy-, 4-Methoxy-, 2-Trifluormethyl- oder 4-Nitro bedeutet.

3. Fungizid, enthaltend einen inerten Trägerstoff und eine fungizid wirksame Menge einer Verbindung der Formel I

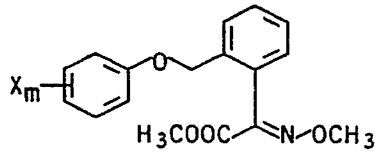

I

in der

X (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,

mit Ausnahme der Verbindungen, bei denen

$X_m$

2-Fluor-, 2-Chlor-, 3-Chlor-, 4-Chlor-, 2,4-Dichlor-, 2-Methyl-4-Chlor-, 2-Methyl-, 4-Methyl-, 4-tert.-Butyl-, 2-Methoxy-, 4-Methoxy-, 2-Trifluormethyl- oder 4-Nitro bedeutet.

4. Glyoxylsäureester der Formel II,

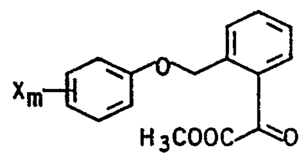

II

in der

X (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,

mit Ausnahme der Verbindungen, bei denen

$X_m$

2-Fluor-, 2-Chlor-, 3-Chlor-, 4-Chlor-, 2,4-Dichlor-, 2-Methyl-4-Chlor-, 2-Methyl-, 4-Methyl-, 4-tert.-Butyl-, 2-Methoxy-, 4-Methoxy-, 2-Trifluormethyl- oder 4-Nitro bedeutet.

5. Mandelsäureester der Formel III,

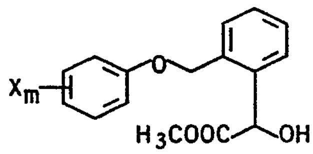

III

in der

X (m = 1 bis 5) gleiche oder verschiedene Substituenten Halogen, Cyano, Nitro,$C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,

mit Ausnahme der Verbindungen, bei denen

$X_m$
2-Fluor-, 2-Chlor-, 3-Chlor-, 4-Chlor-, 2,4-Dichlor-, 2-Methyl-4-Chlor-, 2-Methyl-, 4-Methyl- 4-tert.-Butyl-, 2-Methoxy-, 4-Methoxy-, 2-Trifluormethyl- oder 4-Nitro bedeutet.

6. Benzaldehydcyanhydrine der Formel IV,

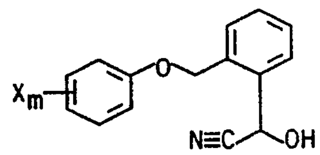

IV

in der
X (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,
mit Ausnahme der Verbindungen, bei denen
$X_m$
2-Fluor-, 2-Chlor-, 3-Chlor-, 4-Chlor-, 2,4-Dichlor-, 2-Methyl-4-Chlor-, 2-Methyl-, 4-Methyl- 4-tert.-Butyl-, 2-Methoxy-, 4-Methoxy-, 2-Trifluormethyl- oder 4-Nitro bedeutet.

7. Benzaldehyde der Formel V,

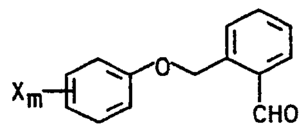

V

in der
X (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,
mit Ausnahme der Verbindungen, bei denen
$X_m$
2-Fluor-, 2-Chlor-, 3-Chlor-, 4-Chlor-, 2,4-Dichlor-, 2-Methyl-4-Chlor-, 2-Methyl-, 4-Methyl-, 4-tert.-Butyl-, 2-Methoxy-, 4-Methoxy-, 2-Trifluormethyl- oder 4-Nitro bedeutet.

8. Oximether gemäß Anspruch 1, dadurch gekennzeichnet, daß $X_m$ 2,4-Dimethyl bedeutet.

9. Oximether gemäß Anspruch 1, dadurch gekennzeichnet, daß $X_m$ 3-Trifluormethyl bedeutet.

10. Verfahren zur Herstellung der Oximether der Formel I

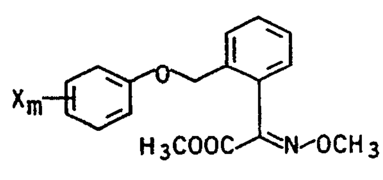

I

in der
X (m = 1 bis 5) gleiche oder verschiedene Substituenten Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder einen gegebenenfalls substituierten annellierten aromatischen Ring bedeutet,
mit Ausnahme der Verbindungen, bei denen
$X_m$
2-Fluor-, 2-Chlor-, 3-Chlor-, 4-Chlor-, 2,4-Dichlor-, 2-Methyl-4-Chlor-, 2-Methyl-, 4-Methyl-, 4-tert.-Butyl-, 2-

Methoxy-, 4-Methoxy-, 2-Trifluormethyl- oder 4-Nitro bedeutet, dadurch gekennzeichnet, daß man ein substituiertes Benzylbromid der Formel VIII

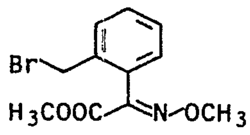

VIII

mit einem substituierten Phenol der Formel

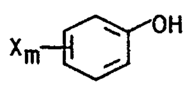

in der $X_m$ die oben genannten Bedeutungen hat, umsetzt.

Europäisches Patentamt

# EUROPÄISCHER RECHERCHENBERICHT

Nummer der Anmeldung

EP 90 10 9441

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| --- | --- | --- | --- |
| D,X | EP-A-0 253 213 (BASF)<br>* Ganzes Dokument *<br>--- | 1-10 | C 07 C 251/40<br>A 01 N 37/50 |
| D,X | EP-A-0 254 426 (ICI)<br>* Ganzes Dokument *<br>----- | 1-10 | |
| | | | **RECHERCHIERTE SACHGEBIETE (Int. Cl.5)** |
| | | | C 07 C 251/00 |

Der vorliegende Recherchenbericht wurde für alle Patentansprüche erstellt

| Recherchenort | Abschlußdatum der Recherche | Prüfer |
| --- | --- | --- |
| DEN HAAG | 02-07-1990 | WELLS A.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)